# EUROPEAN PATENT APPLICATION

(11) **EP 4 686 720 A1**
(43) Date of publication of application: **04.02.2026**
(21) Application number: 24382846.4
(22) Date of filing: 31.07.2024
(51) Int. Cl.: C07D 259/00

(54) **SYNTHESIS OF ORGANIC MACROCYCLES THROUGH CLIP-OFF CHEMISTRY**

(71) Applicant: Fundació Institut Català de Nanociència i Nanotecnologia (ICN2), 08193 Bellaterra (ES); Consejo Superior De Investigaciones Científicas - CSIC, 28006 Madrid (ES); Institució Catalana De Recerca I Estudis Avançats (ICREA), 08010 Barcelona (ES)
(72) Inventor: MASPOCH COMAMALA, Daniel, 08197 VALLDOREIX (ES); IMAZ GABILONDO, Inhar, 08240 MANRESA (ES); SÁNCHEZ NAYA, Roberto, 08208 SABADELL (ES); ALBALAD ALCALÁ, Jorge, 08100 MOLLET DEL VALLÈS (ES); CAVALIERI, Juan Pablo, 08290 CERDANYOLA DEL VALLES (ES)
(74) Representative: ZBM Patents - Zea, Barlocci & Markvardsen

(57) **Abstract**

The present invention refers to a process for preparing a specific organic macrocycle (I), which comprises disassembling a covalent organic framework via clip-off chemistry, which comprises submitting the framework to a bond-breaking reaction to yield a specific macrocycle having equal functional groups. The present invention also refers to the specific organic macrocycles (I₁) - (I₈).

## Description

### Technical Field

The present invention relates to the field of chemical synthesis of compounds, in particular organic macrocycles, and refers to the use of clip-off chemistry as a synthetic strategy to selectively create new molecules and materials, in particular through the programmed disassembly of crystalline reticular structures, in particular covalent organic frameworks.

### Background Art

The synthesis of organic molecules encompasses thousands of methodologies to create new bonds, giving rise to a myriad of compounds, both new and well-known, that find application across various fields, including pharmaceutical, materials science, or agrochemicals. Indeed, organic synthesis excels during the design of small molecules, but struggles to find optimal pathways when dealing with the synthesis of structural materials with elevated degrees of freedom.

A notable example within these challenging compounds is the synthesis of purely organic materials, in particular, organic macrocycles. These molecules have garnered considerable attention because of their highly-preorganized chelating structures, which offer a plethora of fascinating properties and applications in many scientific fields and technologies. Their synthesis, however, presents important challenges, especially on large scales, owing to their intricate structures, presence of coordinating or chelating groups within their bodies, and the need for precise control over cyclization versus polymerization. While various synthetic strategies have been developed that facilitate access to these macrocycles (e.g., templated cyclization, ring-closing metathesis), several limitations still persist. These limitations include low synthetic yields, difficulty in controlling regio- or stereoselectivity, and complex purification and/or separation steps from linear polymeric side-products.

For instance, Johnston, A. G., et al., in "The Synthesis and Solubilization of Amide Macrocycles via Rotaxane Formation", J. Am. Chem. Soc. 1996, 118, 43, 10662-10663, disclose low synthetic yields of organic macrocycles synthetized by traditional means, of only up to 30% yield after purification, since mixtures of products or subproducts are obtained even during the synthesis of simple structures. The method therein used is the formation of rotaxane and its posterior controlled disassembly by transesterification, to obtain simple components. However, this method does not permit selective synthesis of a single compound, hence affecting the obtained yield of the same.

Marti-Centelles, V., et al., in "Macrocyclization Reactions: The Importance of Conformational, Configurational, and Template-Induced Preorganization", Chem. Rev. 2015, 115, 16, 8736-8834 highlight the importance of conformational and configurational preorganization to develop adequate synthetic methods which will enable the obtention of macrocycles in higher yields. In line with this, Han, X.-N., et al., in "Recent advances in the synthesis and applications of macrocyclic arenes", Chem. Soc. Rev., 2023, 52, 3265 review advances made in arene macrocycles synthetic methods, all of which rely of the importance of bond formation.

Most recently, Zalessky, I., et al., in "A Modular Strategy for the Synthesis of Macrocycles and Medium-Sized Rings via Cyclization/Ring Expansion Cascade Reactions", J. Am. Chem. Soc. 2024, 146, 8, 5702-5711 disclose new methods to improve macrocycle synthesis via bond formation, i.e. via kinetically favorable 5 to 7-membered ring cyclizations (CRE cascade reactions) starting from linear precursors, which are then converted into functionalized macrocycles. However, the difficulty in selectively synthetizing macrocycle is also herein disclosed, in that competing intermolecular reactions are inevitable.

Liu, W., et al., in "The Synthesis of a Multiple D-A Conjugated Macrocycle and Its Application in Organic Photovoltaic", Angew Chem Int Ed Engl., Vol. 62, Issue 48, 2023, report moderate to high yield synthesis of organic macrocycles, mostly when the templated effect is optimized for a specific structure, or when the new bond is reversible and allows for defect correction. However, other issues, such as side-product formation and purification steps are still encountered during the synthesis. Thus, none of the methods known in the art enable selective, pure and high yield macrocycle obtention in a simple, scalable, time and cost reducing manner.

Controlled bond-breaking reactions have been disclosed to synthetize different types of molecules or materials. For instance, Yang, Y., et al., in "Clip-off Chemistry: Synthesis by Programmed Disassembly of Reticular Materials", Angew. Chem. Int. Ed. 2022, 61, disclose the programmed disassembly of crystalline reticular structures, such as metal-organic frameworks or metal-organic polyhedra (MOFs/MOPs) through clip-off chemistry. This approach hence permits disassembling MOFs and MOPs to obtain individual 3D structures, wherein a bond between a metal and an organic functional group, such as carboxylate, is formed. Along with the stability provided by their 3D conformation, this metal-organic bond is known to be very robust, rendering MOFs and MOPs with the chemical and thermic stability that characterizes them.

In spite of these advances, synthetizing purely organic molecules composed solely of organic bonds, such as organic macrocycles, in particular those containing reactive functional groups, still remains a challenge by reason of their characteristic reactivity.

Synthesis by direct methods, such as those known in the art, does not enable the formation of macrocycles with reactive groups in high purity and yield, since competing reactions take place and many subproducts are formed. No synthetic method has been described up to date which enables adequate stabilization of organic macrocycles, neither during the process nor once formed, in order to achieve high yield and purity results.

### Summary of Invention

The inventors have surprisingly found that structured purely organic molecules and materials, in particular organic macrocycles, that are typically challenging or impossible to obtain via direct or traditional methods may be effectively synthesized via clip-off chemistry. These molecules have been successfully synthesized in a tailored manner and in short reaction times, giving moreover high selectivity, yield and purity results and thus supposing a great advantage in the field of chemistry.

COFs are robust materials whose main physical properties are driven by strong π-π stacking interactions between their aromatic layered structures. However, chemically, COFs are extended by relatively weak, reversible and dynamic organic bonds, in that they are for instance sensitive to humidity, pH, temperature or nucleophile presence, as opposed to MOFs and MOPs, which, as mentioned above, are formed by strong metal-organic bonds which render them very stable structures. Surprisingly, although the bonds forming the COFs of the present invention are all organic and delocalized bonds, the method of the present invention enables the selective breakage of only one type of bond, whilst maintaining the other type of organic bond unaltered. The inventors have selectively modified specific bonds in the tailored COFs of the present invention to foment the stability of one type of organic bond when submitted to a specific bond-breaking reaction, whilst maintaining the sensitivity of the other type of organic bond to the bond-breaking reaction.

Accordingly, the present invention relates to a process for preparing a specific organic macrocycle (I) which comprises disassembling a covalent organic framework via clip-off chemistry, wherein: the clip-off chemistry technique comprises: submitting the covalent organic framework to a bond-breaking reaction, wherein: the covalent organic framework comprises a dual-pore lattice forming pores by means of both resistant bonds and sensitive bonds to the bond-breaking reaction; the bonds being in a specific position such that, when submitted to the bond-breaking reaction, the resistant bonds enable maintaining the structure of the specific macrocycle forming one of the type of pores, and the sensitive bonds enable isolating the specific macrocycle.

Advantageously, the method of the present invention, in a first step, enables the formation of bonds within the framework, each tailored to meet specific requirements and conditions to reach any target product. More advantageously, the present inventors have surprisingly found that this particular preorganization of the crystalline COF reduces the synthetic degrees of freedom, improving thus the efficiency and predictability of the synthesis of organic macrocycles. Even more advantageously, the synthetic approach of the present invention, *i.e.* the application of clip-off techniques in a second step, surprisingly helps avoiding intermolecular reactions which interfere in both yield and purity of the obtained materials.

Direct synthesis by means of traditional methods does not permit the formation of macrocyclic structures in that competing reactions take place, in particular the formation of simple bonds at end points of the structure formed between the reactants, instead of forming bonds which enable the assembly into a macrocyclic structure. The present invention thus advantageously enables to form a closed structure, in particular a macrocycle, and introduce reactive functional groups, such as aldehydes and carboxylic acids, into the macrocycle after the bond-breaking reaction has been performed. Thus, this reaction selectively takes place in the intended locations of the macrocyclic structure, which translates into high purity and yield results of the specific macrocycles.

Accordingly, a second aspect of the present invention relates to a specific functionalized organic macrocycle (I) obtainable by the process defined above, wherein the macrocycle is selected from the following list, and having the following structure:

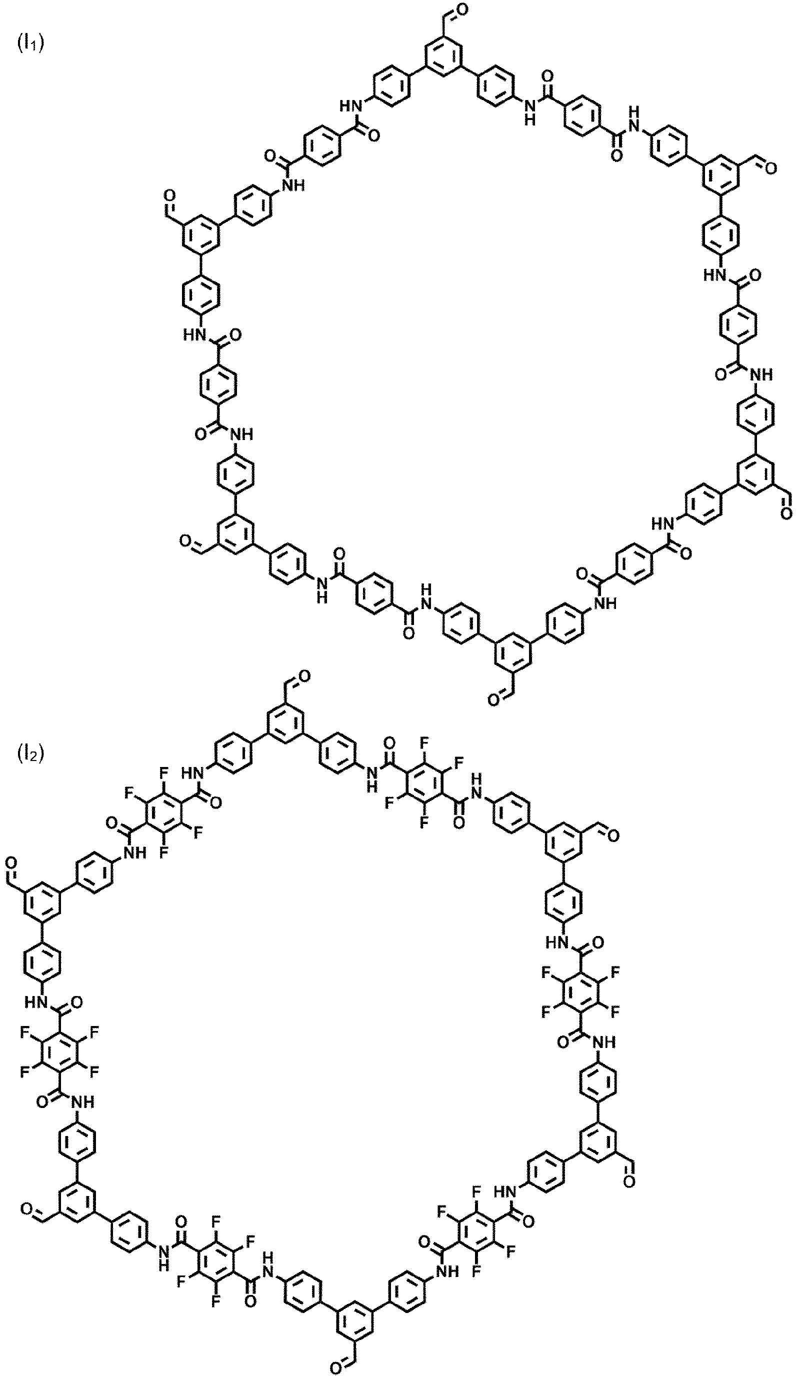

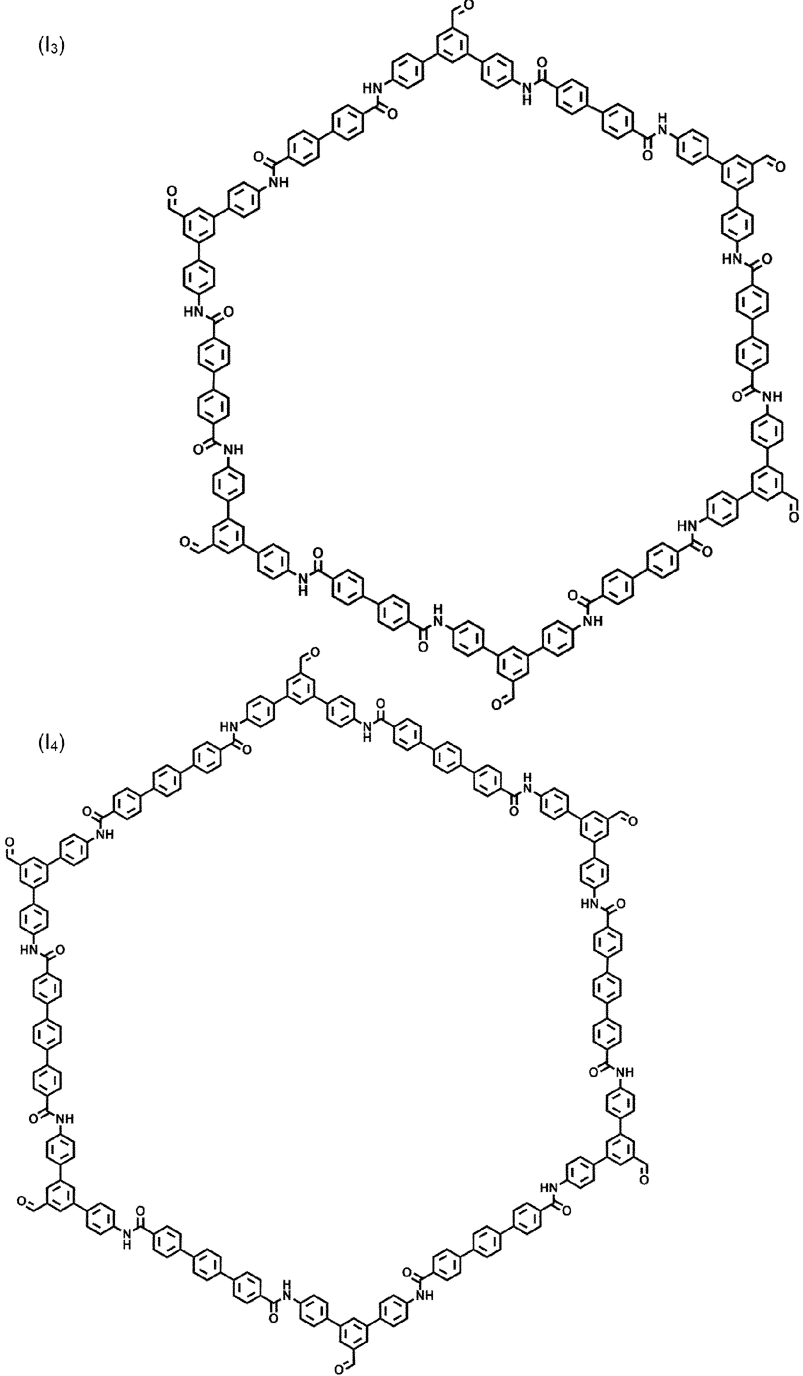

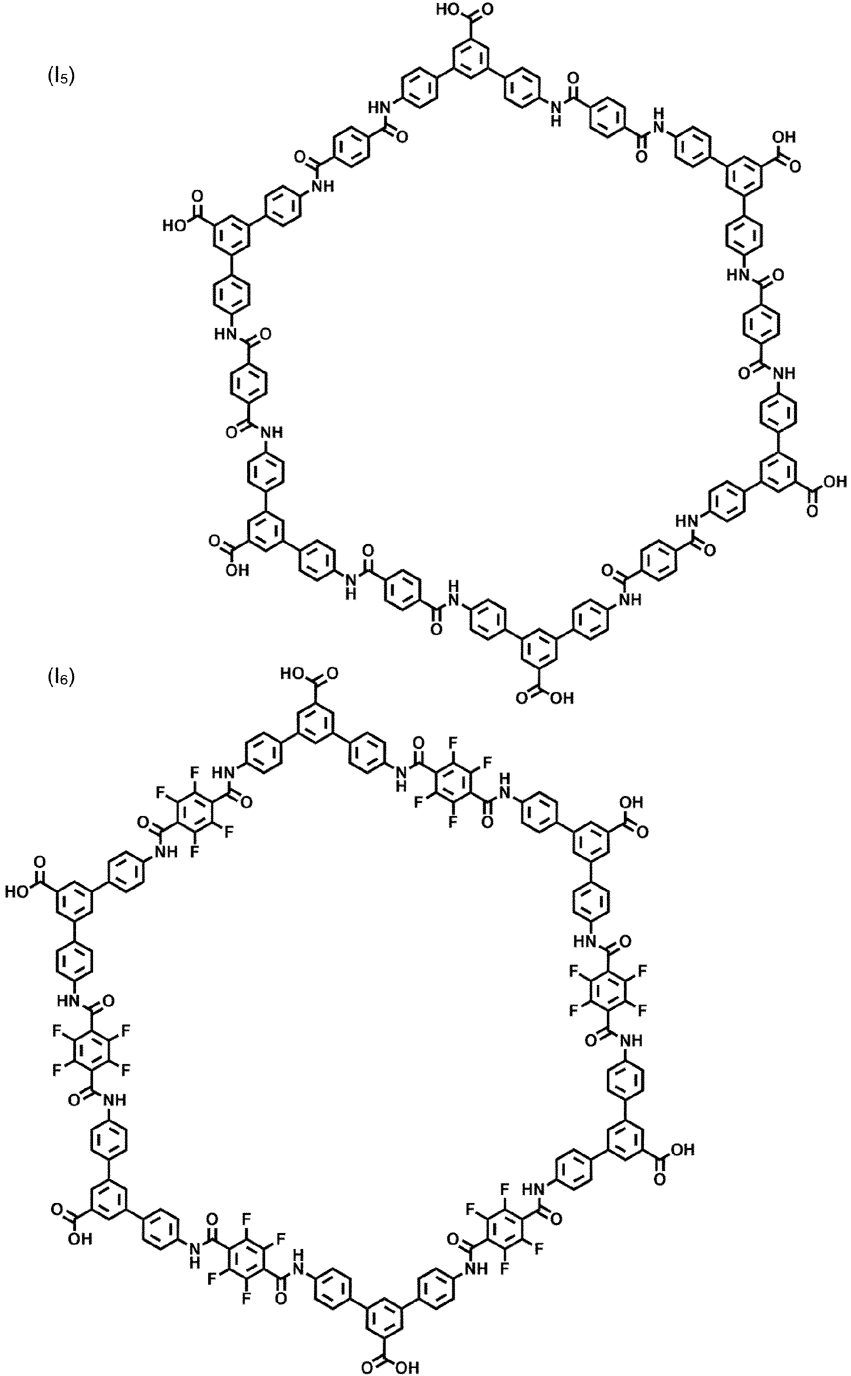

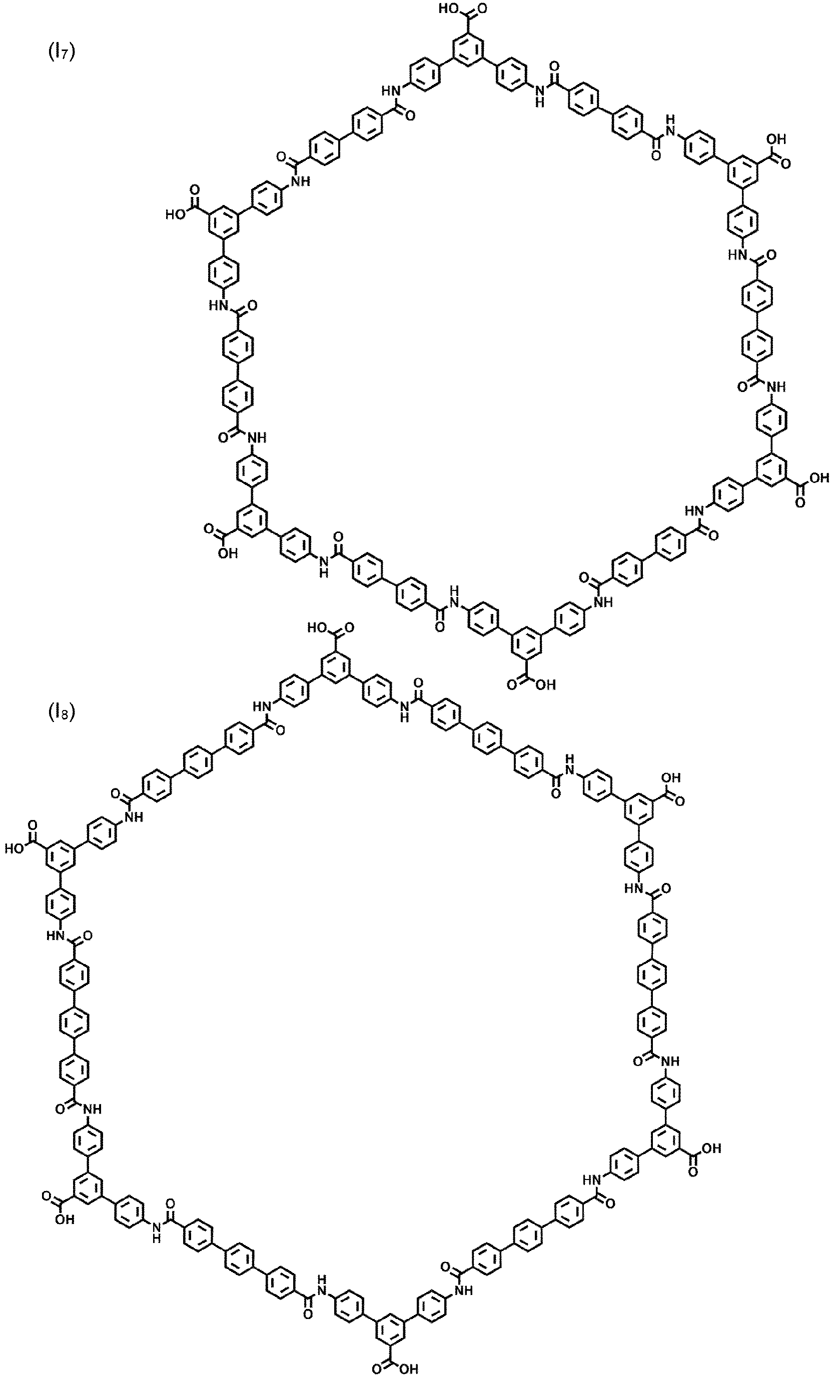

This methodology not only demonstrates the potential for the targeted disassembly of COFs but also opens avenues for the synthesis of novel molecular entities with specific geometries and functionalities derived from the original reticular framework.

### Brief Description of Drawings

Fig. 1 shows a schematic representation of the Clip-off chemistry strategy for the synthesis of novel molecules by selective disassembly of a COF skeleton, where the linear linker (bottom) is condensed by reticular chemistry with the tetratopic building block (top) comprising the cleavable region at its centre.
Fig. 2a shows a scheme of the solvothermal synthesis of the imine-linked COF a) of the present invention.
Fig. 2b shows further oxidation of the imine bonds in the imine-linked COF a) of the present invention to the corresponding amide bonds in the amide-linked COF e) of the present invention, as well as their corresponding XRPD patterns.

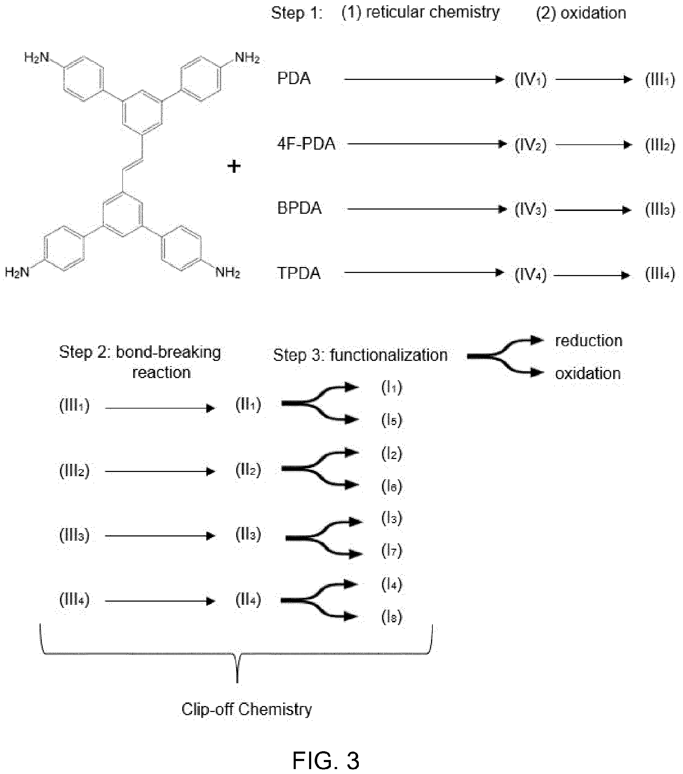

Fig. 3 shows a 3-step synthesis scheme to yield the specific functionalized organic macrocycles (I₁) - (I₈) of the present invention. Step 1 (1) shows the formation of imine-linked reticular materials (IV₁) - (IV₄), which are then submitted to oxidation in Step 1 (2) to form the amide-linked COFs (III₁) - (III₄) of the present invention. The Clip-off Chemistry of the present invention comprises exposing COFs (III₁) - (III₄) to a bond-breaking reaction in Step 2, to obtain products (II₁) - (II₄), and subsequently selectively cleaving products (II₁) - (II₄) in Step 3, either by reduction or oxidation, to isolate the specific functionalized organic macrocycles (I₁) - (I₈).
Fig. 4a shows ¹H NMR spectrum of functionalized macrocycle (I₁) of the present invention (DMSO-*d*₆, 500 MHz) and its comparison with *N,N*'-(5'-formyl-[1,1':3',1"-terphenyl]-4,4"-diyl)dibenzamide (herein referred to as Model-CHO).
Fig. 4b shows ESI-MS spectrum of functionalized macrocycle (I₁) of the present invention.
Fig. 5 shows XRPD pattern of imine-linked COF (IV₁).
Fig. 6 shows Pawley refinement of imine-linked COF (IV₁).
Fig. 7a shows solid-state NMR of (Ills) before and after exposure to ozone.
Fig. 7b shows XRPD of (Ills) before and after exposure to ozone.
Fig. 8 show FTIR spectrum of (III₁) confirming the successful oxidation of the COF linkages, that is from imine to amide bonds.
Fig. 9 shows solid-state NMR analysis of (III₁) confirming the complete transition from imine to amide bonds.
Fig. 10 shows ¹H NMR (500 MHz, DMSO-d₆) of specific aldehyde-functionalized organic macrocycle (I₁).
Fig. 11 shows ¹³C NMR (500 MHz, DMSO-d₆) of specific aldehyde-functionalized organic macrocycle (I₁).
Fig. 12 shows HSQC (500 MHz, DMSO-d₆) of specific aldehyde-functionalized organic macrocycle (I₁).
Fig. 13 shows HMBC (500 MHz, DMSO-d₆) of specific aldehyde-functionalized organic macrocycle (I₁).
Fig. 14 shows DOSY NMR spectrum (500 MHz, DMSO-d₆) of specific aldehyde-functionalized organic macrocycle (I₁).
Fig. 15 shows ESI spectrum (500 MHz, DMSO-d₆) of specific aldehyde-functionalized organic macrocycle (I₂).
Fig. 16 shows ESI spectrum (500 MHz, DMSO-d₆) of specific aldehyde-functionalized organic macrocycle (I₃).
Fig. 17 shows ESI spectrum (500 MHz, DMSO-d₆) of specific carboxylic acid functionalized organic macrocycle (I₅).
Fig. 18 shows ESI spectrum (500 MHz, DMSO-d₆) of specific carboxylic acid functionalized organic macrocycle (I₆).
Fig. 19 shows MALDI spectrum (500 MHz, DMSO-d₆) of specific carboxylic acid functionalized organic macrocycle (I₇).
Fig. 20 shows ¹H NMR spectrum (500 MHz, DMSO-d₆) of specific carboxylic acid functionalized organic macrocycle (I₈).

### Detailed description of the invention

All terms as used herein in this application, unless otherwise stated, shall be understood in their ordinary meaning as known in the art. Other more specific definitions for certain terms as used in the present application are as set forth below and are intended to apply throughout the description and claims.

As used herein, the indefinite articles "a" and "an" are synonymous with "at least one" or "one or more." Unless indicated otherwise, definite articles used herein, such as "the," also include the plural of the noun.

The word "comprise" for the purposes of the present invention encompasses the case of "consisting essentially of" and "consisting of".

Within the context of this specification, "clip-off chemistry" is defined as a controlled bond-breaking reaction which involves the selective cleavage of specific bonds within reticular materials to isolate desired fragments or functionalities, in particular those that are inaccessible by traditional methods.

Within the context of this specification, a "macrocycle" is defined as a large, cyclic molecule of more than 12 atoms, in particular between 12 and 200, which may or not be aromatic rings, and which may or not contain functional groups within its structure.

Within the context of this specification, a "specific macrocycle" is defined as an organic macrocycle structure, product of the process of the present invention, which has been predefined by the specific selection of building blocks, linkers, and bonds to form the covalent organic framework precursor which will be submitted to a clip-off reaction, according to the process of the present invention, isolating thus the specific macrocycle.

For the purpose of the invention, a "functionalized macrocycle" or a "macrocycle having functional groups", which are synonymous in the context of the application and may be used interchangeably, is defined as a macrocycle comprising functional groups, wherein all the functional groups of a given macrocycle equal, for instance, the macrocycle may be functionalized with aldehydes.

The expression "purely organic molecule" refers to a molecule that does not contain any chemical inorganic material, for instance, the COF precursors, as well as the specific macrocycles yielded by the process of the present invention. On the contrary, metal-organic frameworks (MOFs) or polyhedra (MOPs) are examples of metal-organic molecules, in that they include within its organic frameworks and inorganic material, such as a metal.

Within the context of this specification, "reticular chemistry" is defined as the chemistry of linking molecular building blocks by strong bonds to form periodic structures with predefined topologies, namely extended crystalline frameworks.

Within the context of this specification, a "reticular material" is defined as a material that possesses a crystalline nature, thus permitting, for instance, to define the position of every "cleavable bond" within the framework with atomic-level precision. Examples of such materials are covalent organic frameworks (COFs), used within this specification.

Within the context of this specification, "iso-reticular" is defined as having the same or similar structural topology, i.e. the same or similar framework structure, equal directionality and shape. Chemical components and length may be different.

For the purpose of the invention, "*Kagome* topology" or *"Kagome* lattice", which are synonymous in the context of the application and may be used interchangeably, is defined as a two-dimensional lattice pattern of corner-sharing triangles composing a crystal structure. *Kagome* lattice is composed by the combination of trigonal and hexagonal pores.

For the purpose of the invention, "dual-pore lattice" or "dual-pore structure", which are synonymous in the context of the application and may be used interchangeably, is defined as the incorporation of different kinds of pores (micropores, mesopores) into a single polygonal skeleton, forming thus an hetero-pore COF. These kinds if lattices or structures are formed by condensing two different building blocks or linkers. For the purpose of the invention, the symmetric arrangement of monomers is performed via multiple linking sites.

For the purpose of the invention, "linear linker" or "ditopic linker", which are synonymous in the context of the application and may be used interchangeably, is defined as a monomer or linker having 2 binding domains, thus rendering the linker capable of acting as a double-sided connecting node to create a framework. Specifically, they are Ilinear monomers with an adjustable length and that can be coupled with any kind of building block geometries to build 2D or 3D COFs structures.

Within the context of this specification, "aldehyde linear linker" is defined as a linear or ditopic linker in which the binding domains at the terminal position are aldehydes.

For the purpose of the invention, "tetratopic building block" or "tetratopic linker", which are synonymous in the context of the application and may be used interchangeably, is defined as a monomer or linker having 4 binding domains, thus rendering the linker capable of acting as a 4-connecting node and is a successful route to increasing the dimensionality of the assembly. Whereas two building blocks (BBs) that are ditopic form simple structures, larger numbers of end groups, as is the case with tetratopic linkers, can form more complex, polycyclic topologies. The structures and pores of COFs are mainly predetermined through geometric matching of the building units used to form polygonal skeletons. The shapes of the polygons and the pore sizes depend on the geometries and dimensions of the knots and linkers. For example, as mentioned above, condensation of a building unit with a linear linker, or the self-condensation of the C₂-symmetric unit, provides the formation of a hexagonal COF.

The expression "Cₙh" refers to a Cn symmetry axis with n equal to the number of reactive end groups in the building blocks. Since for the purpose of the present invention high symmetry in the BBs is sought, a low number of n is preferred. As such, within the context of this specification, tetratopic C₂h building blocks are preferred. This distinguishes COFs from MOPs, where higher coordination numbers at metal sites may be more easily accessed.

For the purpose of the invention, "cleavable region or site" or "cleavable bond" or "sensitive moiety", which are synonymous in the context of the application and may be used interchangeably, is defined as a precise position within the framework of a reticular material wherein a cleavable bond is found, and hence disassembly of the given reticular material may be pursued by conducting a bond-breaking reaction or technique, such as clip-off chemistry, giving rise to a specific and desired synthetic material. The bond that is to be cleaved will be "sensitive" to the selected bond-breaking reaction, for instance, as is shown in the examples, alkene bonds are sensitive to ozonolysis and thus for this particular reaction these bonds are selected as the "sensitive moiety" defining the "cleavable region" within the reticular material, whereas the "resistant moiety", which are also selectively chosen and strategically positioned within the framework, will not be affected by the reaction, thus enabling the obtention of the specific desired molecule.

Thus, for the purpose of the invention "resistant moiety" or "non-cleaving bonds", which are synonymous in the context of the application and may be used interchangeably, is defined as a bond within a molecule or reticular material which does not change when submitted to the selected bond-breaking reaction or technique. As can be seen in the examples, amide bonds are resistant to ozonolysis and thus for this particular reaction these bonds are selected as the "resistant moiety" which will enable the obtention of the desired product, by maintain its structure. As is the case for the "sensitive moiety" defined above, the "resistant moieties" are also selectively and strategically positioned within the reticular material.

Within the context of this specification, "amide-linked covalent organic framework" is defined as a COF comprising amide bonds within its structure.

Within the context of this specification, "post-synthetic oxidation" is defined as the post-synthetic modification performed on the COF precursor of the present invention in order to modify specific bonds within the matrix, in particular, to oxidize imine bonds to obtain amide bonds. Within the context of this specification, the structure of the COF precursor does not suffer any other significant change due to this modification.

The expression "degrees of freedom" refers to the various ways the atoms within a molecule can move, interact, or rearrange to form different structures and compounds. These englobe multiple factors, such as number of reactive sites, potential stereochemical configurations, or flexibility of molecular chains. In succession, every newly formed bond in a synthetic pathway introduces additional degrees of freedom, increasing exponentially the difficulty of obtaining a desired structural outcome.

As mentioned above, the present invention relates to a process for preparing a specific organic macrocycle (I) which comprises disassembling a covalent organic framework via clip-off chemistry, wherein: the clip-off chemistry technique comprises: submitting the covalent organic framework to a bond-breaking reaction, wherein: the covalent organic framework comprises a dual-pore lattice forming pores by means of both resistant bonds and sensitive bonds to the bond-breaking reaction; the bonds being in a specific position such that, when submitted to the bond-breaking reaction, the resistant bonds enable maintaining the structure of the specific macrocycle forming one of the type of pores, and the sensitive bonds enable isolating the specific macrocycle.

This aspect may also be formulated as the use of clip-off chemistry to perform the disassembly of a covalent organic framework to prepare a specific organic macrocycle (I), wherein: the clip-off chemistry technique comprises: submitting the covalent organic framework to a bond-breaking reaction, wherein: the covalent organic framework comprises a dual-pore lattice forming pores by means of both resistant bonds and sensitive bonds to the bond-breaking reaction; the bonds being in a specific position such that, when submitted to the bond-breaking reaction, the resistant bonds enable maintaining the structure of the specific macrocycle forming one of the type of pores, and the sensitive bonds enable isolating the specific macrocycle.

All the particular embodiments of the process to prepare a specific organic macrocycle (I) by performing the disassembly of a covalent organic framework via clip-off chemistry are also embodiments of the use of the invention.

As mentioned above, organic macrocycles are difficult to synthesize for the above-mentioned reasons, such as degrees of freedom, competition with linear analogues, tedious purification steps to separate the pure product from impurities and/or side products. Thus, the present invention supposes a great advantage in that the tailored formation of a precursor, such as a COF, followed by the controlled disassembly of the same enables the synthesis of these challenging molecules in a simple, short and scalable manner, moreover in high yields and purity.

COFs are majorly synthesized by linking molecular building blocks through dynamic, electronically-delocalized covalent bonds, thus forming a crystalline structure. As defined above, although the chemical components of a reticular material, such as a COF, may differ, its structure, also known as topology, may coincide. Thus, in a particular embodiment of the process according to the invention, in combination with any of the embodiments above or below, the covalent organic framework is iso-reticular.

COFs are crystalline porous organic structures with well-defined two- or three-dimensional (2D or 3D) structures composed of building blocks being connected via covalent bonds, forming periodic skeletons and ordered pores and hence differentiating them from other types of porous materials, such as MOFs. They are designed and synthesized by combining monomers, also called linkers, according to the intended topology. Thus, in a particular embodiment of the process according to the invention, in combination with any of the embodiments above or below, the covalent organic framework is two-dimensional (2D). The abundant possibilities in designing pore environment in COFs enable richer and wider possibilities in their further structural modifications when compared to their counterparts, for instance, MOFs. Moreover, tailored COFs of the present invention (III) are particularly advantageous when compared to other organic polymers in that their crystalline structure enables determining the specific location of its components and/or bonds.

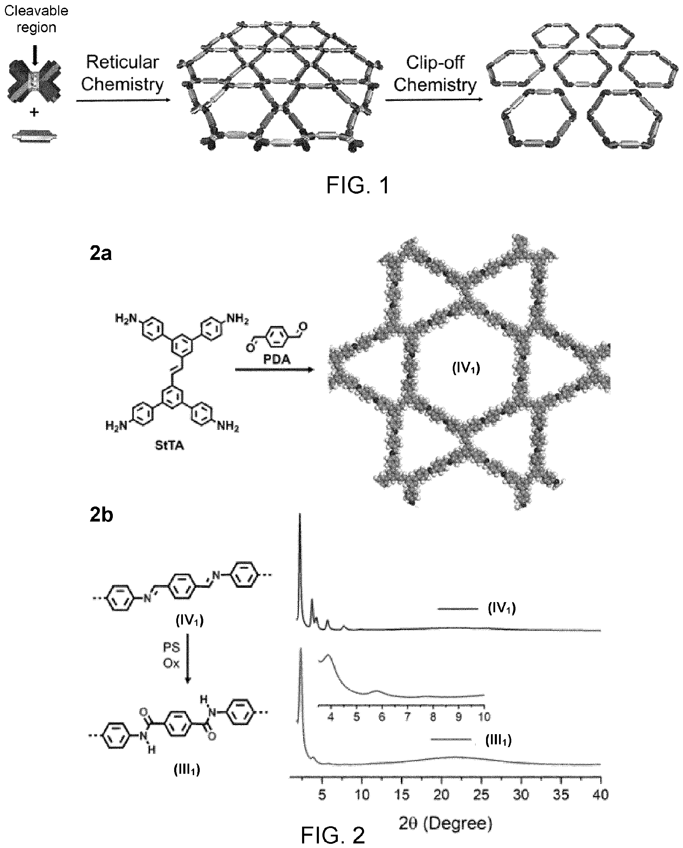

As defined above, *Kagome* topology is a two-dimensional lattice pattern of corner-sharing triangles, forming thus a mother framework that contains two distinct types of pores (also known as "dual-pore lattice"), that is, a periodic assembly of alternating hexagonal and trigonal pores (see FIG. 1). COFs with kgm (*Kagome*) topology are commonly obtained by combination of a tetratopic C₂h building block with a linear one. The hexagonal pores are exclusively extended by the condensation linkages formed during COF synthesis, and those are interconnected through the bisection of the C₂h building block. Moreover, the inventors have tailored the COF precursor by strategically and advantageously making the central part of the C₂h linker the only cleavable site within the COF, thus enabling the preparation of isolate hexagonal macrocycle species (i.e. the target molecule) upon clip-off chemistry, as can also be seen in FIG.1.

Thus, in a particular embodiment of the process according to the invention, in combination with any of the embodiments above or below, the covalent organic framework is a 2D framework with dual-pore lattice. In a more particular embodiment, in combination with any of the embodiments above or below, the dual-pore lattice forms a 2D *Kagome* topology composed of hexagonal and trigonal pores.

2D COFs are synthesized by the co-condensation of monomers to form building blocks which will eventually form a crystalline structure. As mentioned above, the present inventors have surprisingly and advantageously found that COF precursors with *Kagome* topology enables the selective synthesis of purely-organic molecules, in particular hexagonal macrocycles. As mentioned above, dual-pore *Kagome* lattice is composed of the condensation of two different linkers or monomers. Accordingly, in a particular embodiment of the process according to the invention, in combination with any of the embodiments above or below, the dual-pore lattice is composed of combinations of tetratopic linkers and linear linkers. In a more particular embodiment, in combination with any of the embodiments above or below, the tetratopic linkers are tetratopic C₂h linkers and the linear linkers are aldehyde linear linkers.

In an embodiment of the process of the present invention, clip-off chemistry enables the selective cleavage of an X - Y bond, as shown below, wherein the X-Y bond can be selected from a single, double, or triple bond. In a particular embodiment of the process of the present invention, clip-off chemistry is performed selectively on the X-Y bond, that is the sensitive moiety, without affecting the A-B bond, that is, the resistant moiety which, according to the present invention, has been selectively introduced during the assembly of the reticular precursor (in this case, COF), or has been transformed, for example, by post-synthetic oxidation.

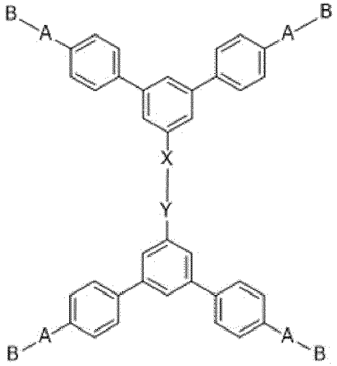

As shown in the examples, the present inventors have used (E)-3,3',5,5'-tetrakis(4-aminophenyl) stilbene (StTA) as the C₂h linker. This compound is a monomer with a central cleavable site (as mentioned and indicated below) when exposed to the specific bond-breaking reaction of the present invention, namely an alkene bond, and has the following structure:

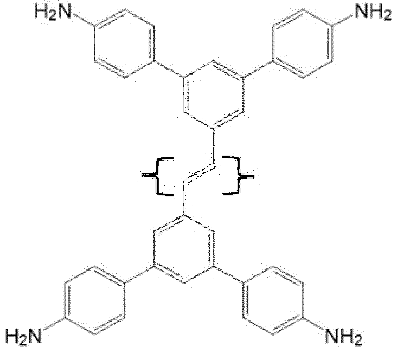

Thus, in a particular embodiment of the process according to the invention, in combination with any of the embodiments above or below, the covalent organic framework comprises cleavable regions. Accordingly, in a more particular embodiment of the process according to the invention, in combination with any of the embodiments above or below, the *Kagome* lattice is composed of combinations of C₂h linkers which are strategically and advantageously made the only cleavable site within the COF.

In another embodiment of the process according to the present invention, in with any of the embodiments above or below, C₂h linkers comprising a cleavable site X-Y as shown above are selected from the following structures:

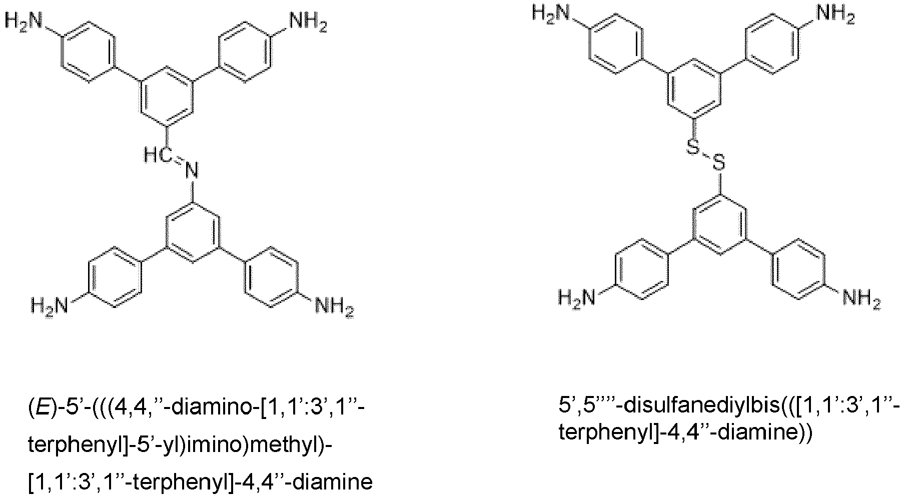

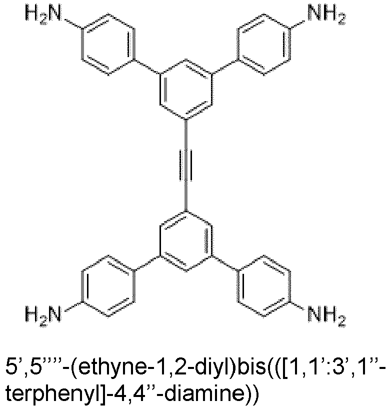

As shown in the Examples, the building blocks in the COF precursor of the present invention are formed by condensation of StTA and a linear linker, namely an aldehyde linker, selected from the group consisting of benzene-1,4-dicarboxaldehyde (PDA), tetrafluoroterephthalaldehyde (4F-PDA), [1,1'-Biphenyl]-4,4'-dicarbaldehyde (BPDA), and [1,1':4',1"-Terphenyl]-4,4"-dicarboxaldehyde (TPDA), having the following structures:

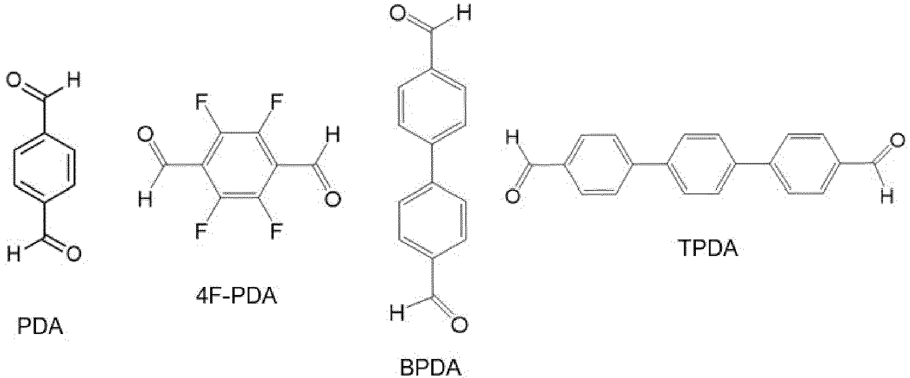

Aldehyde linkers are preferred in that they advantageously enable the formation of a highly crystalline structure, which is particularly relevant to tailor the COF precursors (III) of the invention. Thus, in a particular embodiment of the process according to the invention, in combination with any of the embodiments above or below, the tetratopic C₂h linkers are selected from the group consisting of (E)-3,3',5,5'-tetrakis(4-aminophenyl) stilbene, (*E*)-5'-(((4,4,"-diamino-[1,1':3',1"-terphenyl]-5'-yl)imino)methyl)-[1,1':3',1"-terphenyl]-4,4"-diamine, 5',5ʺʺ-disulfanediylbis(([1,1':3',1"-terphenyl]-4,4"-diamine)), and 5',5""-(ethyne-1,2-diyl)bis(([1,1':3',1"-terphenyl]-4,4"-diamine)), and the aldehyde linear linkers are selected from the group consisting of benzene-1,4-dicarboxaldehyde (PDA), tetrafluoroterephthalaldehyde (4F-PDA), [1,1'-Biphenyl]-4,4'-dicarbaldehyde (BPDA), and [1,1':4',1"-Terphenyl]-4,4"-dicarboxaldehyde (TPDA).

In another particular embodiment of the process according to the invention, in combination with any of the embodiments above or below, the tetratopic C₂h linker is (E)-3,3',5,5'-tetrakis(4-aminophenyl) stilbene, and the aldehyde linear linker is selected from the group consisting of benzene-1,4-dicarboxaldehyde (PDA), tetrafluoroterephthalaldehyde (4F-PDA), [1,1'-Biphenyl]-4,4'-dicarbaldehyde (BPDA), and [1,1':4',1"-Terphenyl]-4,4"-dicarboxaldehyde (TPDA).

Macrocycles with varied lengths can be achieved by elongating the dialdehyde building blocks. Inner diameters of the pores in the formed COFs will increase when implementing longer linkers, whether the linear or the tetratopic linkers or both are elongated, as shown in Example 6.

The condensation product resulting from the combination of StTA building blocks and PDA, 4F-PDA, BDPA, or TPDA aldehyde linkers (as shown in Example 1) is an extended imine-linked framework with kgm (*Kagome*) topology, in the present specification referred to as (IV). The structures of the four different imine-linked COFs are shown below, wherein i) is a general representation of the COF structure, that is a lattice formed by the combination of hexagonal and trigonal pores, and wherein ii) is a closer view of the length of the chains (depending on the aldehyde linker) as well as the bonds forming the pores, that is the alkene bond (sensitive moiety) strategically placed at the periphery of the hexagonal pores, and the imine bond (which will suffer a post-synthetic transformation to obtain the resistant moiety) forming the core of the hexagonal pore. 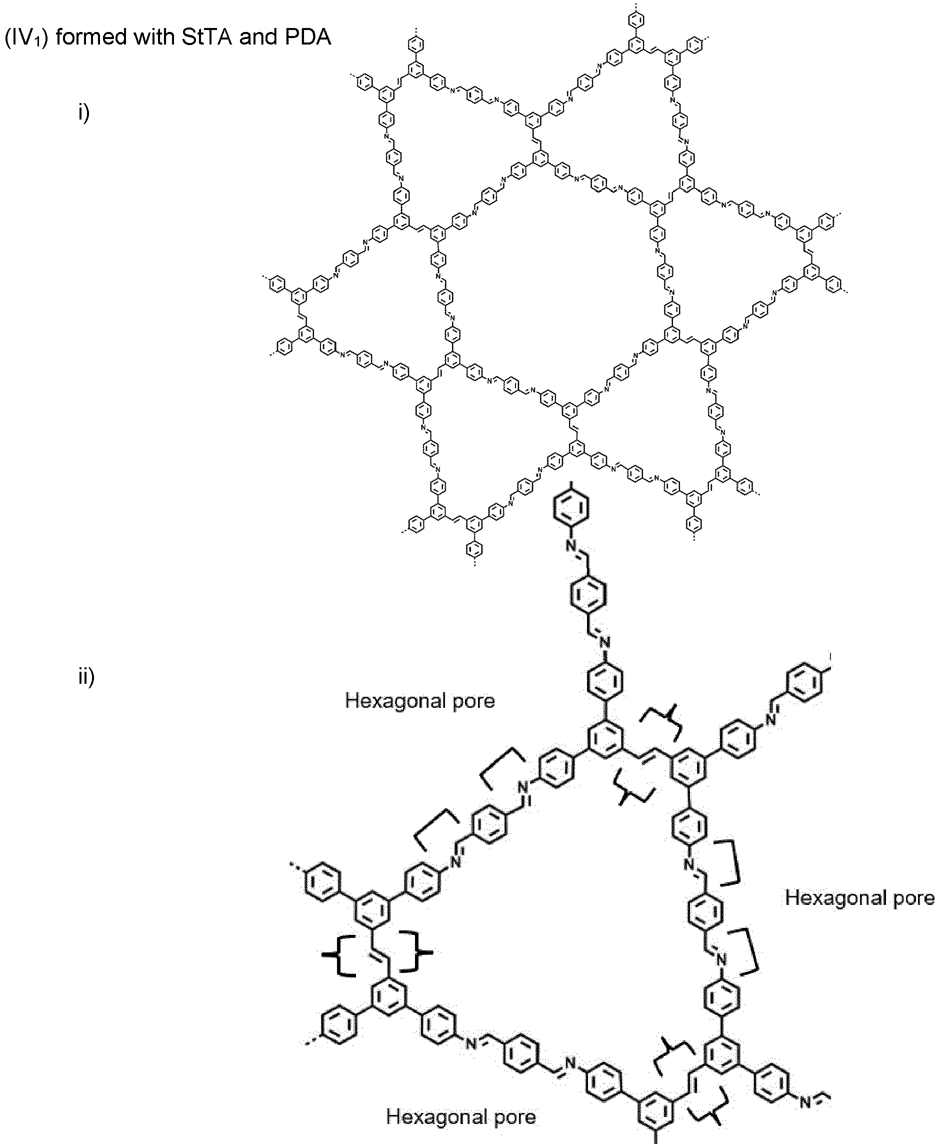 (IV₂) formed with StTA and 4F-PDA 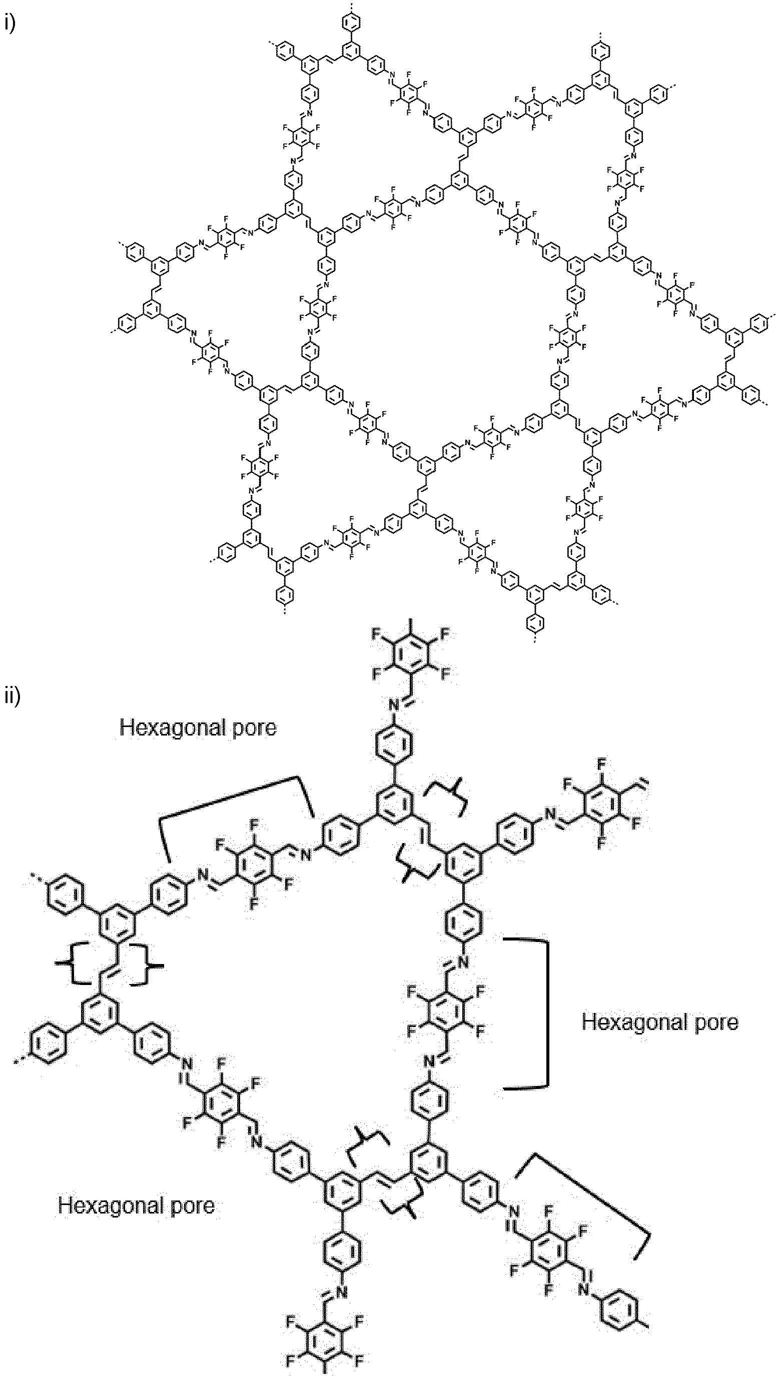 (IV₃) formed with StTA and BPDA 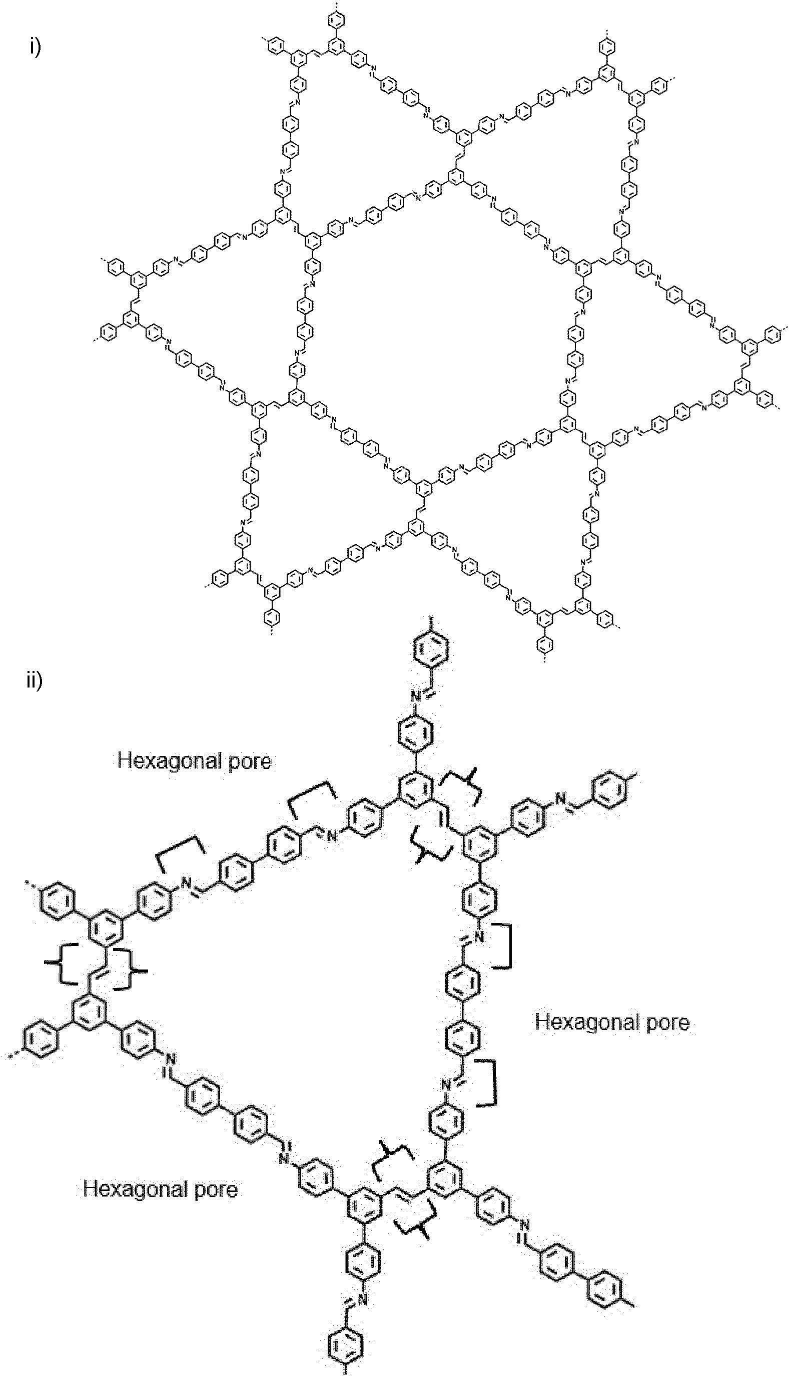 (IV₄) formed with StTA and TPDA

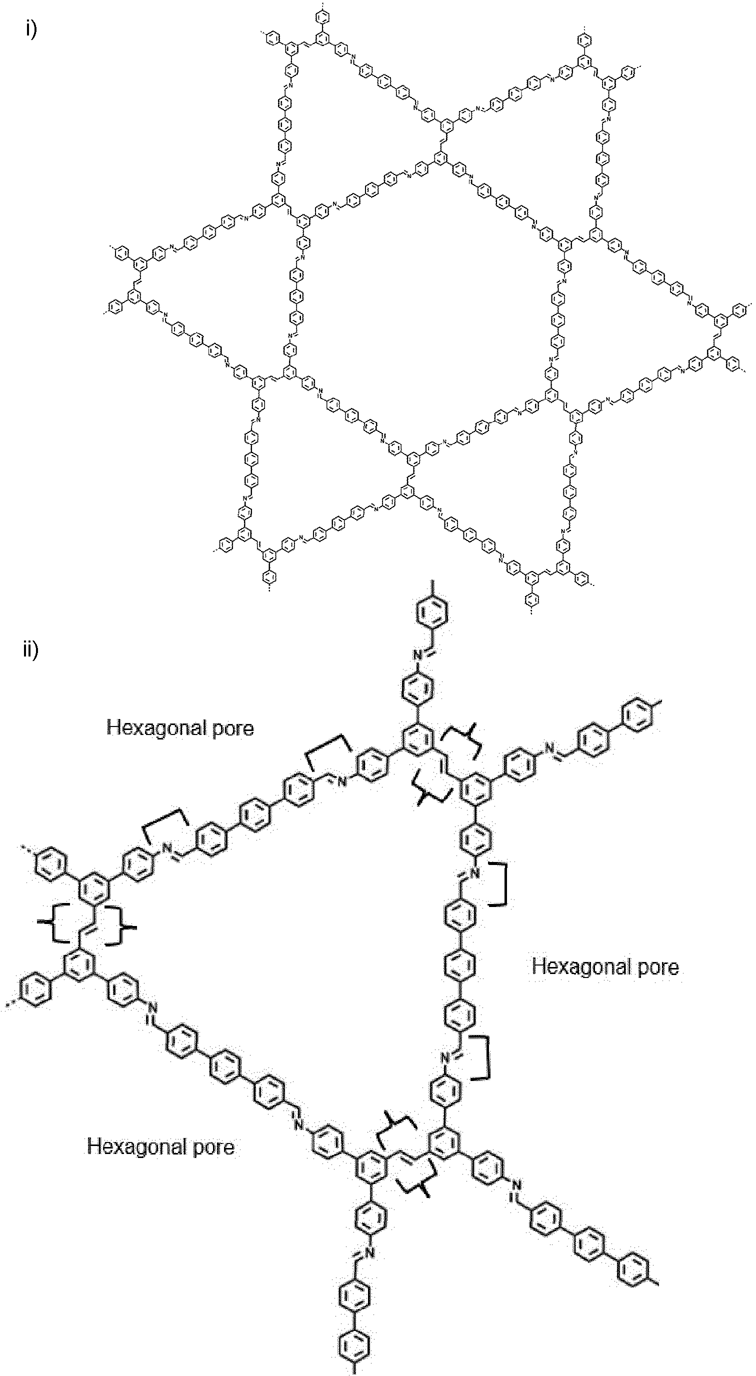

As mentioned, COFs are formed by covalent bonds, which are particularly prone to react with moisture, nucleophiles, or oxidizing agents. This is, for instance, the case for imine (C=N) bonds, the most representative linkages in COF chemistry, which are formed in the COF precursors of the present invention, as shown in Example 1. These bonds, for instance, are known to readily dissociate in the presence of ozone into both nitro and carbonyl or carboxylate fragments.

Clip-off chemistry is based on using, as starting materials, structures that contain cleavable groups at specific positions, such that cleavage of said groups generates new molecules or materials. As such, it encompasses different types of controlled bond-breaking reactions, including thermolysis, photolysis, enzymatic cleavage, or chemical bond-breaking reactions which enable selectively targeting and cleaving specific sections within a molecule or material. Thus, in a particular embodiment of the process according to the invention, in combination with any of the embodiments above or below, the bond-breaking reaction is selected from the list consisting of ozonolysis, photothermal cleavage, redox cleavage, and pH-triggered cleavage. In a more particular embodiment, the redox cleavage is KMnO₄₋ cleavage, and the pH-triggered cleavage is acid cleavage. In an even more particular embodiment, when the COF structure is submitted to photothermal cleavage, the yielded macrocycle comprises thiol (SH₂) functional groups. In an even more particular embodiment, when the COF structure is submitted to KMnO₄₋ cleavage, the yielded macrocycle comprises carboxylic acid (COOH) functional groups. In an even more particular embodiment, when the COF structure is submitted to acid-cleavage, the yielded macrocycle comprises a mixture of amine (NH₂) and aldehyde (CHO) functional groups. In a more particular embodiment of the process according to the invention, the sensitive moieties are introduced into the COF structure as part of the C₂h building block.

In a particular embodiment of the process according to the invention, in combination with any of the embodiments above or below, the bond-breaking reaction is ozonolysis, and the moieties are ozone-resistant bonds and ozone-sensitive bonds. In a more particular embodiment of the process according to the invention, the sensitive moiety is selected from the group consisting of an alkene bond, and an alkyne bond. In another more particular embodiment of the process according to the invention, when the bond-breaking reaction is ozonolysis, the resistant moiety is selected from the group consisting of amide bond, imide bond, and boronic ester. In an even more particular embodiment of the process according to the invention, the ozone-resistant moiety is an amide bond, and the ozone-sensitive moiety is an alkene bond.

In another particular embodiment of the process according to the invention, in combination with any of the embodiments above or below, when the bond-breaking reaction is ozonolysis, the process further comprises a previous step which comprises submitting the imine-linked covalent organic framework to an oxidation reaction to yield an amide-linked covalent organic framework. In a more particular embodiment, the alkene bond is introduced into the COF structure as part of the StTA building block.

In another particular embodiment of the process according to the invention, in combination with any of the embodiments above or below, when the bond-breaking reaction is ozonolysis, the process further comprises a subsequent step which comprises performing an oxidation or reduction reaction to yield a specific organic macrocycle (I) having aldehyde or carboxylic acid functional groups, respectively.

In another more particular embodiment, when the bond-breaking reaction is acid cleavage, the sensitive moiety is an imine bond. In another more particular embodiment, when the bond-breaking reaction is photocleavage, the sensitive moiety is a disulfide bond. In another more particular embodiment, when the bond-breaking reaction is KMnO₄ cleavage, the sensitive moiety is an alkyne bond.

Post-synthetic modification or functionalization of imine-based COFs, for example COFs for CO₂ capture, may be synthesized by post-synthetic modification and functionalization of imine-based structures.

In a particular embodiment of the process according to the invention, a post-synthetic oxidation to an imine-linked COF precursor (IV) of the present invention is performed, to obtain an amide-linked COF (III), in view of achieving stable, resistant bonds. Advantageously, the amide bonds within an extended COF matrix show stability when exposed to a constant ozone flux, on the contrary to imine bonds which underwent complete dissolution, as shown in Example 2.

Thus, as shown in Fig. 3, step 1 (2), in a particular embodiment, a post-synthetic oxidation on the imine-linked COF precursors (IV) of the present invention is performed (as shown in Example 3) to obtain the desired ozone-resistant amide-linked COF precursors of the present invention, referred to as (III) in the present specification. The structures of the four different amide-linked COFs are shown below, wherein i) is a general representation of the COF structure, that is a lattice formed by the combination of hexagonal and trigonal pores, and wherein ii) is a closer view of the length of the chains (depending on the aldehyde linker) as well as the bonds forming the pores, that is the alkene bond (sensitive moiety) strategically placed at the periphery of the hexagonal pores, and the amide bond, that is, the resistant moiety forming the core of the hexagonal pore which will ultimately enable the release of the macrocycle, product of the present invention.

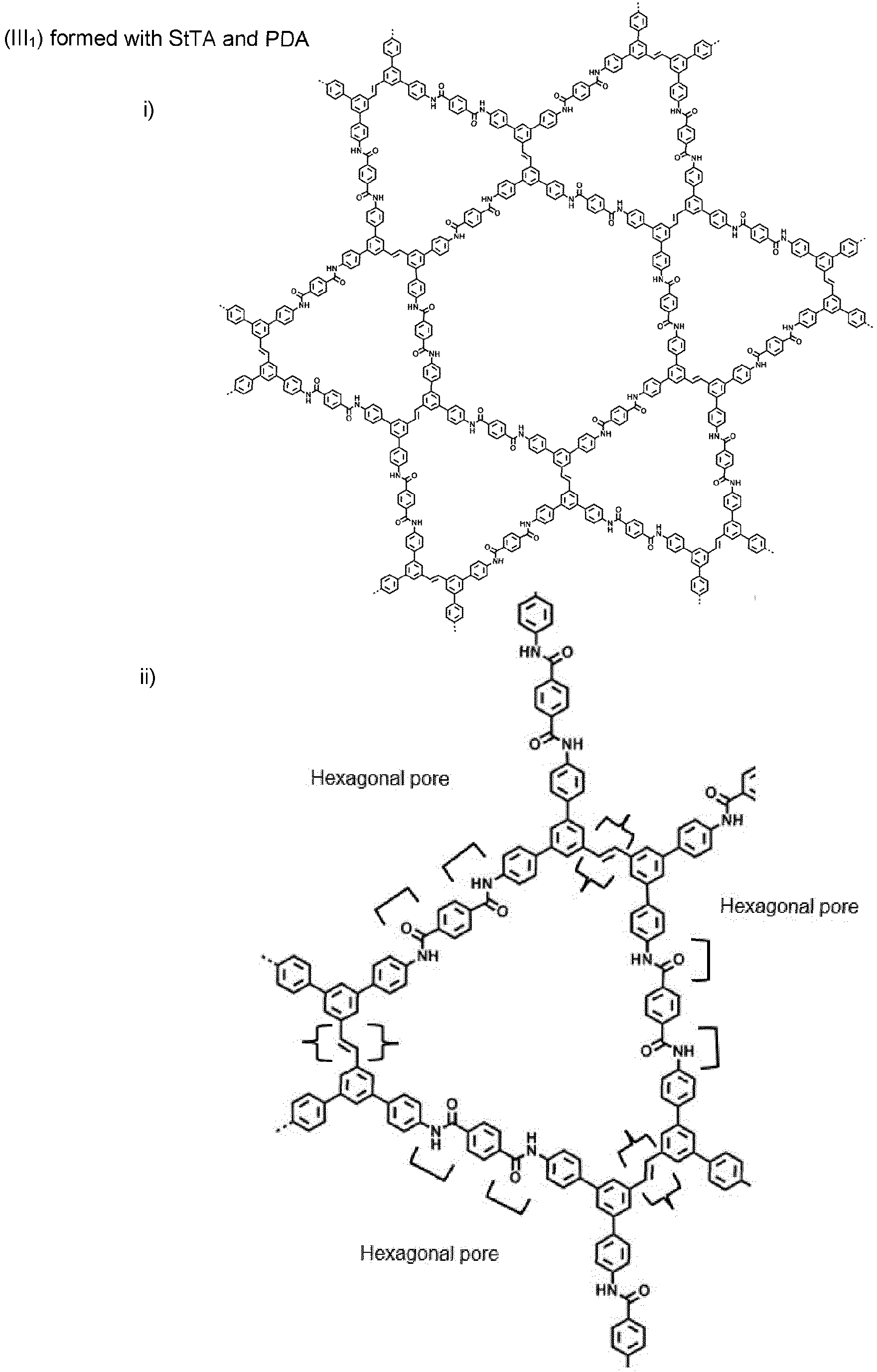

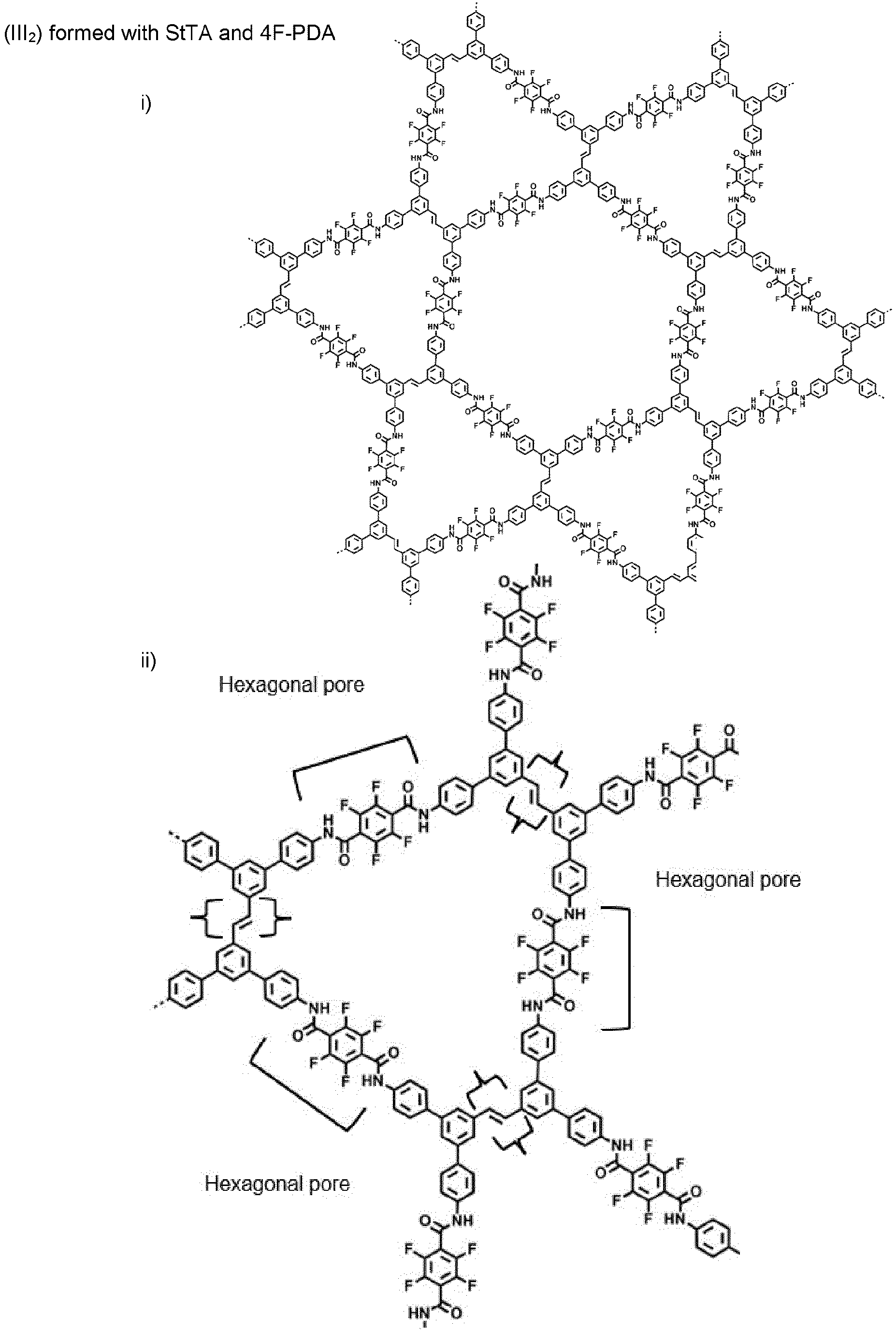

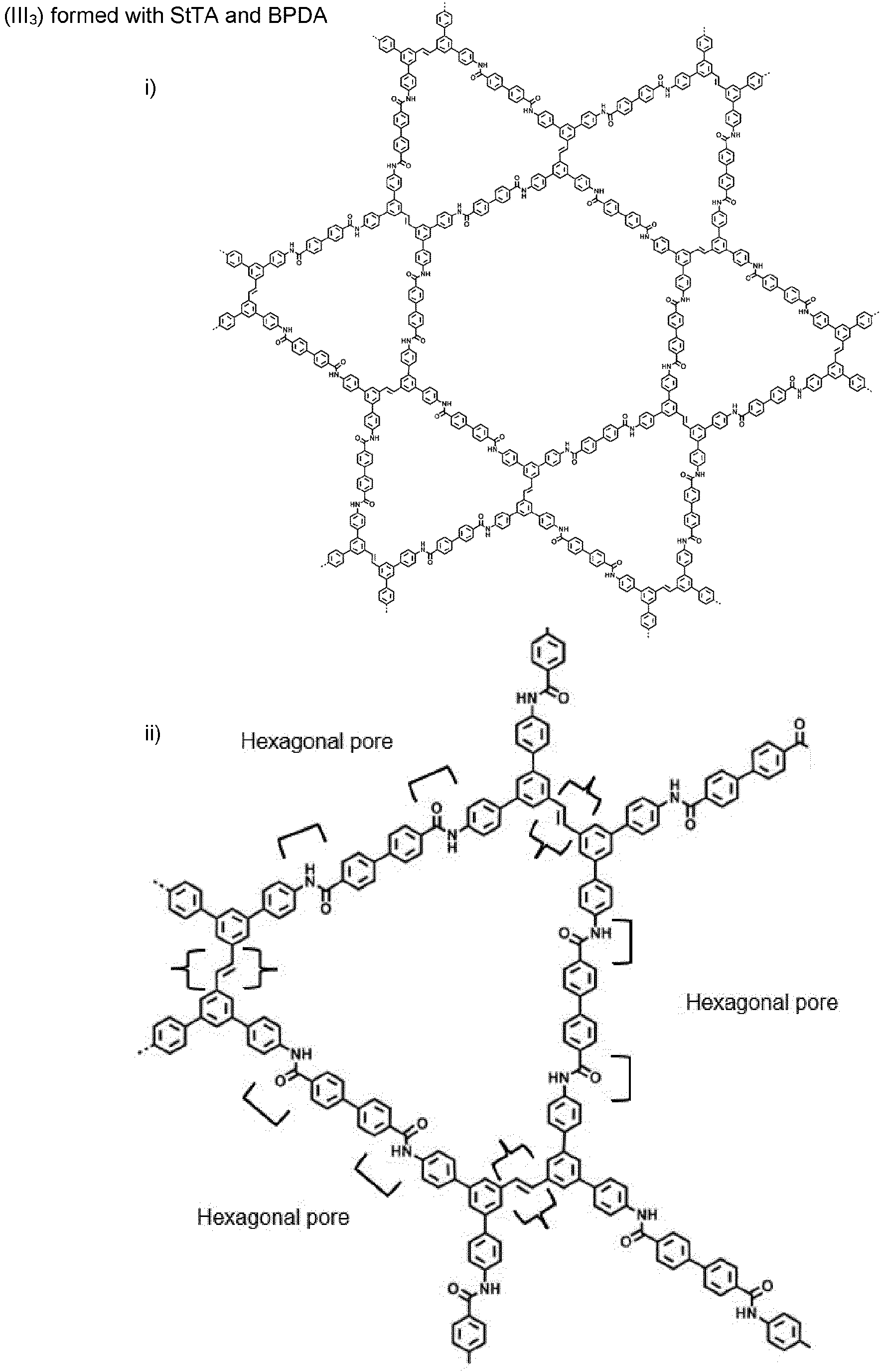

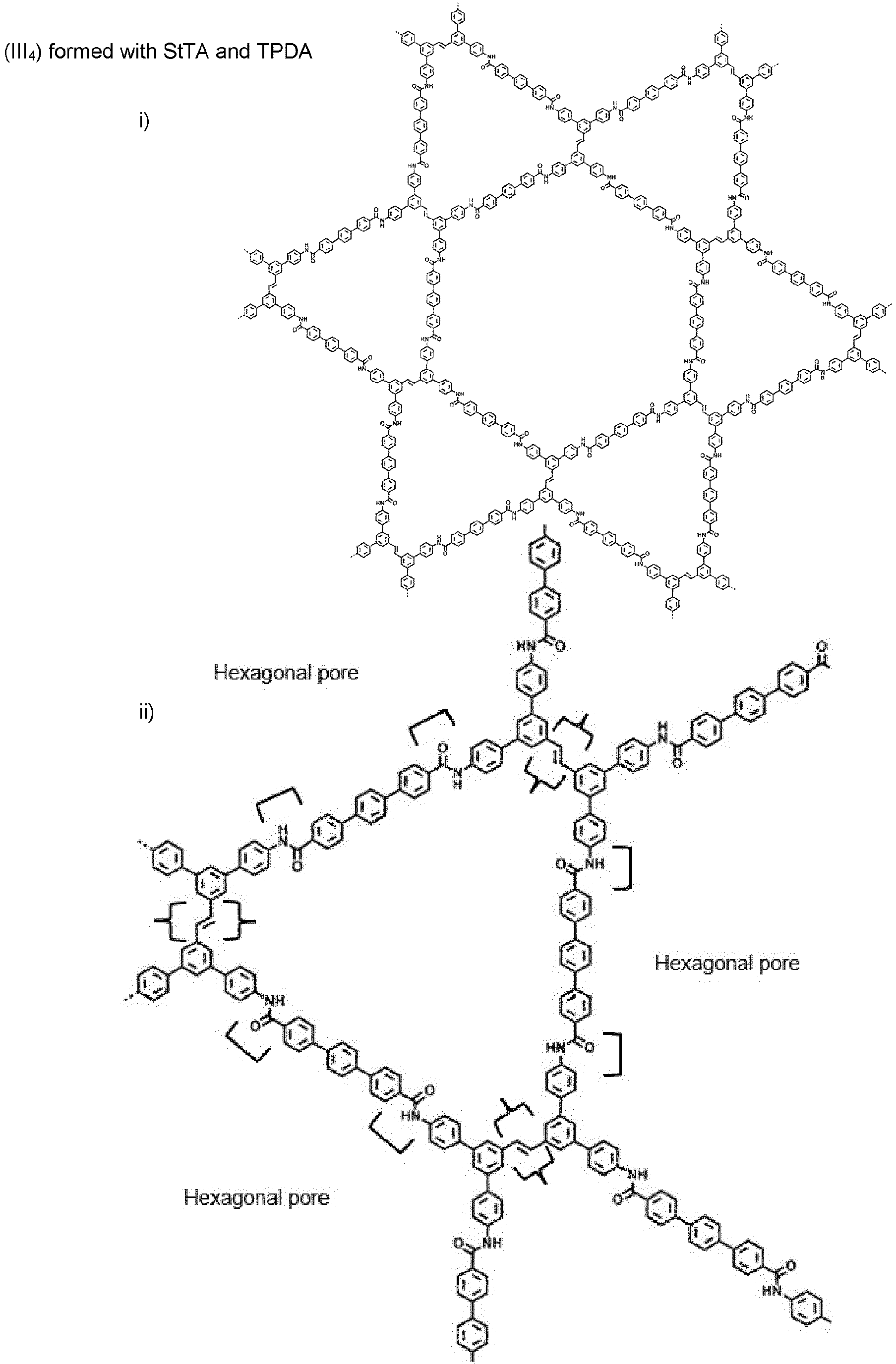

The process of the present invention allows tailoring a COF precursor which includes strategically designed building blocks that, when combined, create a pore without cleavable sites within its ring. The other adjacent pore must interlink the former hexagonal pores through cleavable bonds at its periphery. This tailored design developed ensures that upon exposure of the COF precursor to a specific bond-breaking reaction, the target molecules (formed by the first pore) can be exclusively isolated as discrete molecular entities, due to the dissolution of the other pores with cleavable regions.

Thus, by forming the COF dual-pore lattice in a first step and oxidizing the obtained COF precursor in a second step, the process of the present invention represents an advantage in that the resistant bonds are strategically positioned within the hexagonal pores of the COF matrix, which are intended to be isolated to give as a result the desired purely-organic macrocycles. Thus, in a particular embodiment of the process according to the invention, in combination with any of the embodiments above or below, the covalent organic framework is composed of hexagonal and triagonal pores formed by a combination of PDA and StTA, wherein the StTA comprises the only cleavable region of the framework, and wherein the hexagonal pores are embedded with ozone-resistant amide bonds. In an embodiment of the process according to the invention, in combination with any of the embodiments above or below, the disassembly of the COF via clip-off chemistry enables the isolation of the hexagonal pores from the COF matrix.

In a particular embodiment, the ozonolysis conditions are advantageously applied to an amide-linked COF precursor, which has an aromatic core where both amide and alkene bonds coexist simultaneously.

In another embodiment of the process according to the invention, in combination with any of the embodiments above or below, the ozonolysis reaction is carried out in an appropriate solvent or solvent combination, wherein the dispersion is bubbled at a temperature ranging from -90 to 25 °C, and with a concentration of between 0.5 and 3 g/h of ozone. In a more particular embodiment of the process according to the invention, in combination with any of the embodiments above or below, the solvent combination is a mixture of organic solvents in which ozone and the molecular products obtained by ozonolysis are soluble. In a more particular embodiment of the process according to the invention, in combination with any of the embodiments above or below, the solvent combination is a mixture of DMF/MeOH, and a co-solvent selected from the group consisting of THF, DCM, chloroform, or ethyl acetate. In a more particular embodiment, in combination with any of the embodiments above or below, the dispersion is bubbled for a for a period of time between 5 and 60 minutes. In a more particular embodiment, in combination with any of the embodiments above or below, the dispersion is bubbled at a temperature of -78°C for 10 minutes, and with a concentration of 3 g/h of ozone.

As shown in FIG. 3 steps 2 and 3, comprising applying the clip-off chemistry process of the present invention to the COF precursor, eight specific organic macrocycles (I) with hexagonal structure have been yielded. Thus, in a particular embodiment of the process according to the invention, in combination with any of the embodiments above or below, the covalent organic framework is disassembled into hexagonal subunits. In a more particular embodiment of the process according to the invention, in combination with any of the embodiments above or below, the hexagonal pores in the *Kagome* lattice are isolated to obtain the desired hexagonal molecular macrocycles. In another particular embodiment of the process according to the invention, in combination with any of the embodiments above or below, the organic macrocycle is an amide-linked macrocycle.

In another embodiment of the process according to the invention, in combination with any of the embodiments above or below, the comprises the following steps:
(a) synthetizing a covalent organic framework precursor comprising cleavable regions via reticular chemistry, as defined above,
(b) performing direct synthesis of amide bonds within the covalent organic framework obtained in (a) or post-synthetic oxidation of the imine bonds comprised within the framework obtained in (a) to form resistant amide bonds, as defined above,
(c) exposing the oxidized covalent organic framework precursor obtained in (b) to an ozonolysis reaction as defined above,
(d) submitting the covalent organic framework obtained in (c) to a further oxidation or reduction reaction to obtain a functionalized macrocycle with aldehyde or carboxylic acid functional groups, respectively, as defined above, and
(e) stirring the resultant products for a time period between 120 and 4320 minutes (72 h), removing the solvent, redissolving the obtained solid in a different solvent and precipitating the product.

In a more particular embodiment of the process according to the invention, in combination with any of the embodiments above or below, the solvent used to redissolve the solid is selected from the list consisting of DMSO, DMF, NMP, MeOH, and acetone. In a more particular embodiment of the process according to the invention, in combination with any of the embodiments above or below, the product is precipitated in ethyl acetate.

The organic macrocycles obtained via clip-off chemistry of the present invention have a very high purity and yield. Furthermore, as can be seen in the Examples, in addition to the possibility of synthesizing in a continuous way and extremely reducing reaction times compared to traditional methods, it is highly advantageous that no work-up steps are needed to obtain the product with high purity, since no intermolecular reactions take place and hence no by-products are produced.

This is particularly surprising and advantageous for the preparation of macrocycles containing highly reactive functional groups, such as aldehyde and carboxylic acid, which tend to be involved in intermolecular reactions, giving rise to by-products, thus reducing purity and yield of the final desired product. The examples illustrate eight different functionalized organic macrocycles, referred to as (I₁) - (I₈) in the present specification, all of which are amide-linked, and of which four are functionalized with aldehydes and the other four with carboxylic acids.

The specific organic macrocycles (I₁) - (I₈) of the invention are advantageously obtained by the process of the present invention, in which COFs (III₁) - (III₄), when submitted to ozonolysis, yield to products (II₁) - (II₄), and which are subsequently submitted to an oxidation or reduction reaction to yield specific macrocycles (I₁) - (I₈), as shown in FIG. 3 steps 2) and 3).

Thus, in a particular embodiment of the process according to the invention, in combination with any of the embodiments above or below, the subsequent reduction reaction is performed in the presence of DMS, Zn/HCl, pyridine, or PPh3, and at a temperature ranging from -80°C to 25 °C. In a more particular embodiment, the reduction reaction starts at a temperature of -78°C and is left for a period of time ranging from 1 to 8 hours until it arrives to 25°C.

In another particular embodiment of the process according to the invention, in combination with any of the embodiments above or below, the subsequent oxidation reaction is performed with an excess Oxone^{®} at a temperature ranging from 20 to 30 °C. In a more particular embodiment, in combination with any of the embodiments above or below, temperature for the oxidation reaction is 25 °C.

Thus, a second aspect of the present invention refers to a specific functionalized organic macrocycle (I) obtained by the process of the present invention as defined above, wherein the macrocycle is selected from the following list: (I₁), (I₂), (I₃), (I₄), (I₅), (I₆), (I₇), and (I₈).

The present inventors have surprisingly implemented clip-off chemistry in purely organic materials, that is a covalent organic framework, in a successful manner. This is further considered surprising and advantageous in that the study of a transition from a reticular system (solid state) to a soluble molecular entity poses significant challenges, especially when the solid precursor cannot easily be digested, as is the case, since the COF precursor has been post-synthetically modified to introduce resistant amide bonds.

Throughout the description and claims the word "comprise" and variations of the word, are not intended to exclude other technical features, additives, components, or steps.

Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Abbreviations

COF (covalent organic framework), kgm (*Kagome*), StTA ((E)-3,3',5,5'-tetrakis(4-aminophenyl) stilbene), Am (amide), Im (imine), TAPB (tris(4-aminophenyl)benzene), PDA (terephthalaldehyde or benzene-1,4-dicarboxaldehyde), tetrafluoroterephthalaldehyde (4F-PDA), [1,1'-Biphenyl]-4,4'-dicarbaldehyde (BPDA), [1,1':4',1"-Terphenyl]-4,4"-dicarboxaldehyde (TPDA), hcb (hexagonal close-packed), DMF (dimethylformamide), MeOH (methanol), 2D (bi-dimensional), 3D (tri-dimensional), ¹HNMR (Proton Nuclear Magnetic Resonance), ESI (electro-spray ionization), ESI-MS (electro-spray ionization mass spectrometry), DMS (dimethyl sulfide), DMSO (dimethyl sulfoxide), DMSO-d₆ (deuterated dimethyl sulfoxide), THF (tetrahydrofuran), ¹³C NMR (Carbon-13 nuclear magnetic resonance), HSQC (Heteronuclear single quantum coherence spectroscopy), HMBC (Heteronuclear Multiple Bond Coherence), FTIR (Fourier-transform infrared spectroscopy), XRPD (X-ray powder difraction), DOSY NMR (Diffusion-Ordered NMR Spectroscopy).

### Examples

### Example 1: COF precursor formation

A first imine-linked COF precursor (IV₁) was synthesized with a combination of (E)-3,3',5,5'-tetrakis(4-aminophenyl) stilbene (StTA) as the selected C₂h building block and terephthalaldehyde (PDA) as the linear linker to form the *Kagome* lattice of the crystalline structure.

The kgm series of (IV₁) - (IV₄) have been synthesized using an adapted method from Dichtel, W.R., et al., in "Single-Crystalline Imine-Linked Two-Dimensional Covalent Organic Frameworks Separate Benzene and Cyclohexane Efficiently", Journal of the American Chemical Society 2022, 144 (43), 19813-19824.

In a 20 mL scintillation vial, benzoic acid (114 mg) were immersed in benzonitrile (2 mL). The vial was heated to 65 °C until the dissolution of the solid. To the vial, 250 µL of a stock solution of the respective aldehyde building block (PDA: 24 mg/mL to obtain (IV₁), 4F-PDA: 36.9 mg/mL to obtain (IV₂), BPDA: 37.4 mg/mL to obtain (IV₃), TPDA: 51 mg/mL to obtain (IV₄)) were added. Subsequently, water (60 µL) and aniline (40 µL of a 0.7 M solution in benzonitrile) were added and the mixture was gently mixed. Finally, 250 µL of a stock solution of StTA (54.5 mg/mL). The vial was capped and heated to 85 °C for 72 hours. The precipitate was filtered and washed with acetone and MeOH several times, then further washed by Soxhlet extraction in MeOH overnight. The collected sample was activated by exchanging the solvent with liquid CO₂ and passing a continuous flow of supercritical CO₂ for two hours to afford imine-linked COFS (IV₁) - (IV₄) of the present invention as bright yellow or orange solids (Yields: 80-90%).

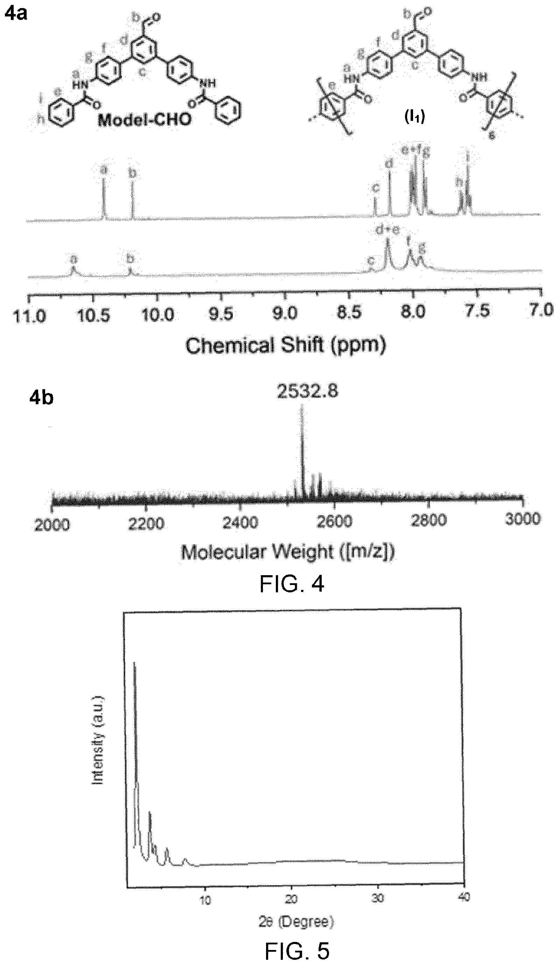

COF (IV₁) was obtained in a 95% yield by using a slightly modified protocol reported by Dichtel, W.R., *et. al.* StTA and PDA were co-condensed in a scintillation vial for three days at 85 °C in the presence of benzoic acid as a catalyst and aniline as a structural modulator. Powder X-ray diffraction (XRPD, FIG. 5) measurements revealed sharp and intense peaks at 2.21 °, 3.75°, 4.29°, 5.67°, and 7.64°, indicating the formation of a highly crystalline structure with the expected kgm topology with an eclipsed AA stacking (FIG. 2b).

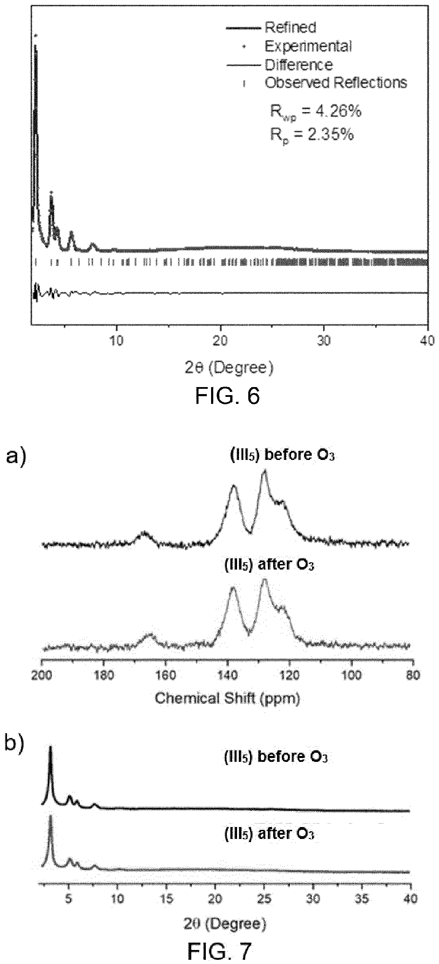

Pawley refinement of COF (IV₁) was performed, and the following parameters were obtained: lattice parameters of a=b = 49.33 Å, and c = 3.53 Å, with angles of α = β = 90° and γ = 120° were obtained (FIG 6). The fact that differences between the "experimental" and the "refined" COF are barely observed in FIG. 6 confirms the high degree in purity and crystallinity of the tailored COF. Thus, practically no amorphous zones or structural defects are detected, meaning that no sub-products are to be expected after ozonolysis is applied to the COF.

### Example 2: Ozone stability of amide bonds within an extended COF matrix

To confirm the ozone stability of amide bonds within an extended COF matrix, two isostructural COF analogs, (IV₅) and (III₅), extended through imine and amide bonds respectively, were subjected to a constant ozone flux. The structure of both COFs (IV₅) and (III₅), comprised a 2D hexagonal close-packed (hcb) topology with hexagonal pores, resulting from the combination of the trigonal tris(4-aminophenyl) benzene (TAPB) and the linear terepthalaldehyde (PDA) building blocks. The structure represents the ideal organic scaffold to study the effect of exposing solid COFs to ozone and decipher its effect on both their physical and chemical properties. When a dispersion of (IV₁) was exposed to ozone in a mixture of DMF/MeOH, the material underwent complete dissolution in seconds due to the uncontrolled fragmentation of the COF skeleton. On the contrary, (III₅) obtained through quantitative post-synthetic oxidation of the former imine-linked COF, did not suffer any significant degradation after being exposed to ozone for hours, as evidenced by solid-state NMR (FIG. 7a) and XRPD (FIG. 7 b).

### Example 3: Oxidation of imine-linked COFs (IV₁) - (IV₄) into amide-linked COFs (III₁)(III₄)

The oxidation of imine-linked COFs (IV₁) - (IV₄) into amide-linked COFs (III₁) - (III₄) was carried out by treating the solid with sodium chlorite and acetic acid in the presence of 2-methyl-2-butene and dioxane.

20 mg of the corresponding imine-linked COF were introduced in a 4.5 ml vial. Then dioxane (2 mL) and 2 methyl-2-butene (100 eq. per imine group) were added. Next, an aliquot of a 3.3 M aqueous solution of NaClO₂ was introduced (10 eq. per imine group), followed by glacial AcOH (10 eq. per imine group). The vial was covered in aluminum foil to avoid the light and left rotating at 15 rpm for 24 hours. After this time, another aliquot of NaClO₂ 3.3 M was added (10 eq. per imine) and the vial was left rotating for another 24 hours. Upon completion of this time, the solid was filtered and washed with H₂O, aqueous Na₂S₂O₃ (10% w/w), H₂O, dioxane, THF, acetone and MeOH. The collected sample was activated by exchanging the solvent with liquid CO₂ and passing a continuous flow of supercritical CO₂ for two hours, to afford amide-linked COFs as pale brown solids (Yields: 90-100%, probably due to the presence of adsorbed water molecules).

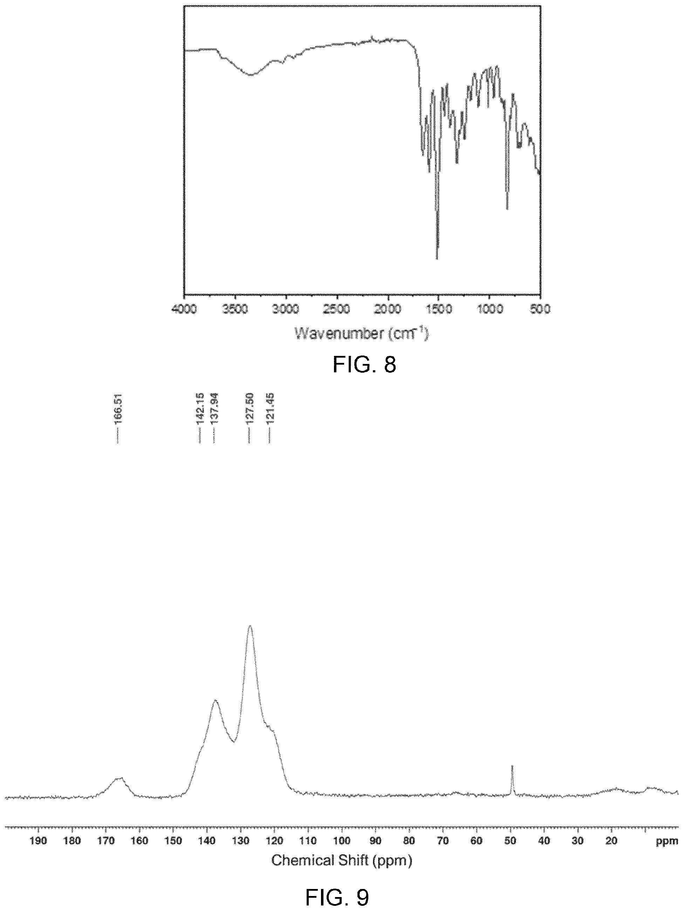

The complete fading of the C=N imine stretch (1620 cm⁻¹) and the subsequent emergence of C=O amide stretch signals (1656 cm⁻¹) in the FTIR spectrum of (III₁) confirmed the successful oxidation of the COF linkages (FIG. 8). Additionally, the complete transition from imine to amide bonds was unambiguously confirmed by solid-state NMR analysis, where the carbon signals corresponding to the amide groups appeared at 166.5 ppm, while the signals associated with the imine groups at 157.7 ppm were simultaneously attenuated (FIG. 9). Moreover, the initial sharp and intense reflection at 2.31° in the XRPD pattern indicated that the product retained its crystalline nature without significant changes on its long-range kgm distribution (FIG. 2b).

### Example 4: Clip-off chemistry applied to amide-linked COFs to obtain aldehyde-functionalized organic macrocycles

The present inventors exposed (III₁) - (III₄) precursors to reductive ozonolysis to obtain (I₁) - (I₄) functionalized macrocycles. The ozonolysis was carried on a solvent combination that favors the solubility of the formed discrete products. At the same time, to achieve a single product, the intermediate macrocycle generated during the ozonolysis reaction underwent a reductive work-up at a temperature of -78 °C to ensure phase purity into aldehyde groups upon fragmentation.

Thus, (III₁) - (III₄) were dispersed in a mixture of DMF/MeOH using THF as a co-solvent to avoid the freezing of DMF, while simultaneously ensuring the solubilization of the formed macrocycles. The dispersion was bubbled at -78 °C for ten minutes with a concentration of 3 g/h of ozone. Smoothly, the solid material dissolved even after just one minute. Upon completion of the time, dimethyl sulfide (DMS) was added to ensure the reductive conditions. After stirring the resultant products for two hours at 25°C, the solvent was removed and the obtained solid was redissolved in dimethyl sulfoxide (DMSO) and precipitated with ethyl acetate.

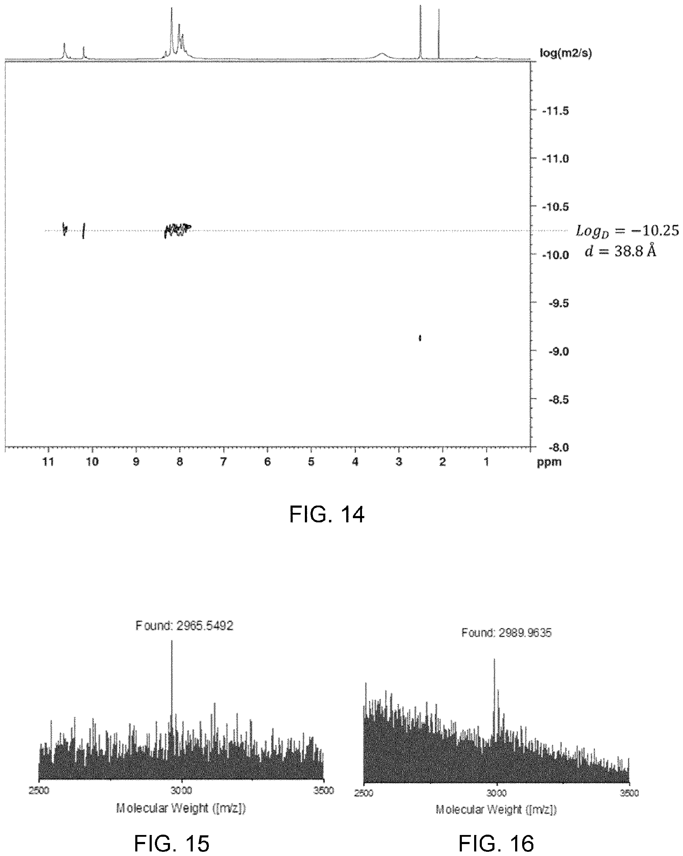

10 mg of the corresponding amide-linked COF (III₁) - (III₄) were dispersed in a mixture of DMF/THF/MeOH (Vt = 2 mL, ratio 0.8/0.8/0.4 mL for (III₁) and (III₂), and 0.9/0.9/0.2 mL for (III₃) and (III₄)) for ten minutes using an ultrasonication bath. The dispersion was cooled down to -78 °C and connected to a custom-made ozone generator. The dispersion was subsequently bubbled with ozone gas (Flow: 1 L/min, C: 50 g/N m³, approx. 3 g/h) for ten minutes. Upon completion of this time, the resulting solution was bubbled with air for 5 minutes and 200 µL of DMS (FIG. 3, step 3, reduction) were injected to the solution. The solution was left stirring at room temperature for two hours. The solution was centrifuged to remove minor solid traces and then the solvent was taken and evaporated under reduced pressure. The obtained solid was redissolved using hot DMSO, then precipitated with EtOAc and washed with EtOAc, acetone and dried. The resulting solid was finally stirred overnight in aqueous Na₂CO₃ (5 g/L), washed with water, acetone, and dried under high vacuum at 85 °C overnight to yield the corresponding aldehyde-functionalized macrocycles (I₁) - (I₄) as beige solids: 15 mg of (I₁), 8 mg of (I₂), 7.5 mg of (I₃), 1.5 mg of (I₄). The resultant solids were further washed with aqueous sodium carbonate and dried under high vacuum. FIG. 15 and 16 show ESI spectrums (500 MHz, DMSO-d₆) of specific functionalized organic macrocycles (I₂) and (I₃).

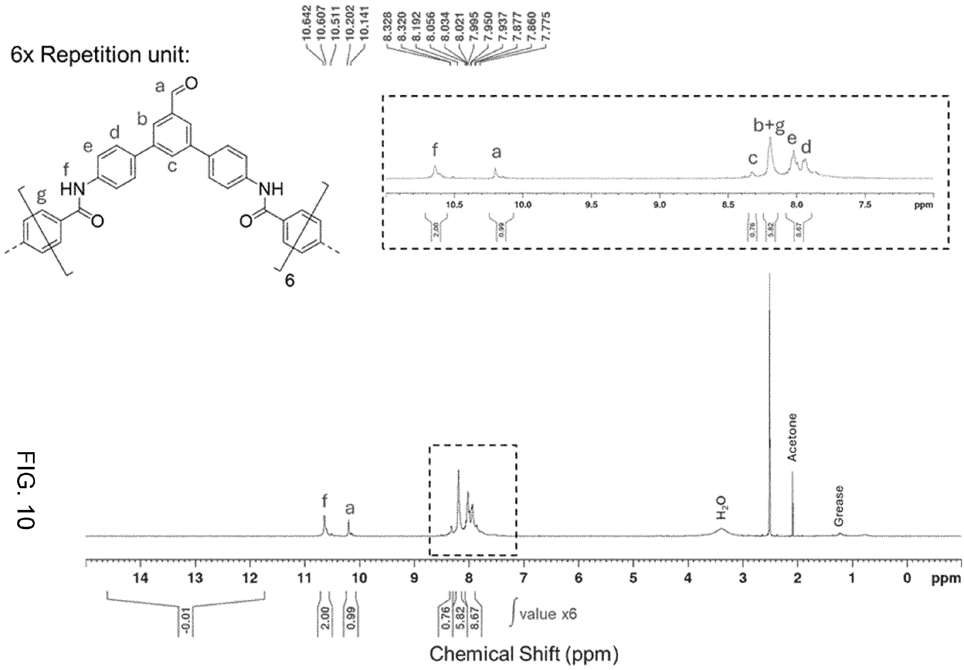

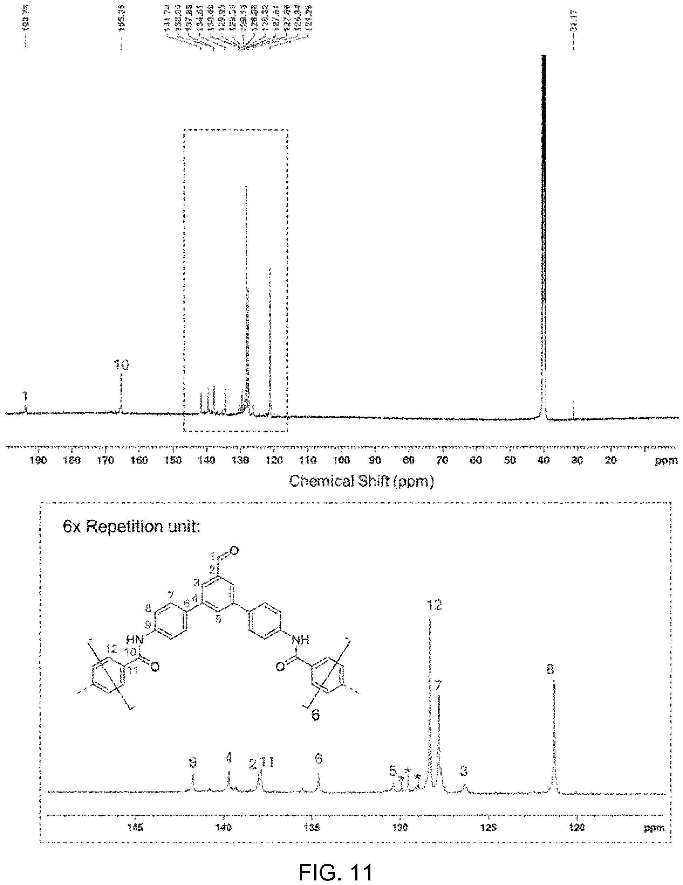

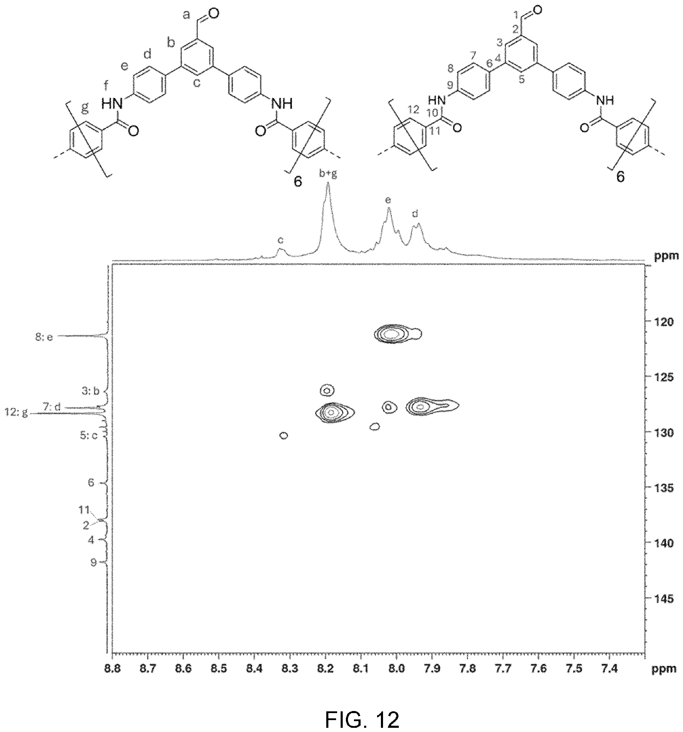

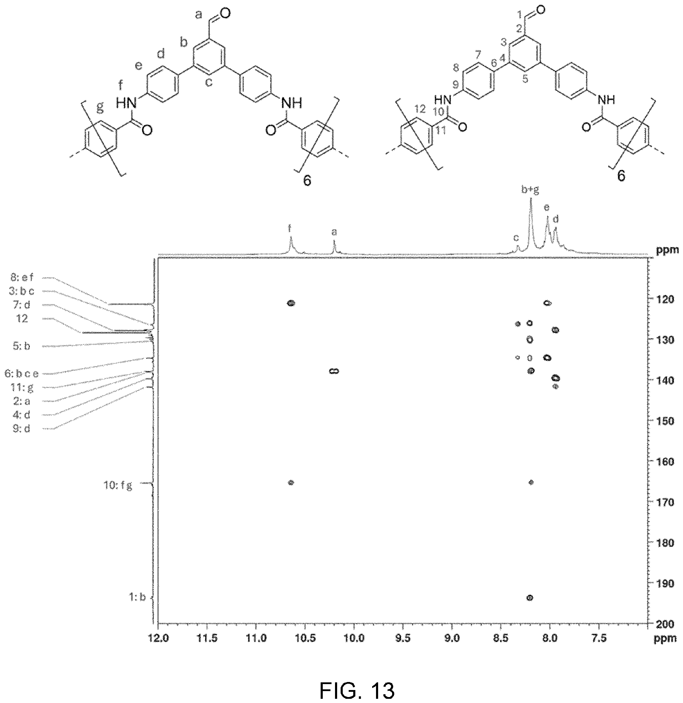

An initial ESI-MS of product (I₁) revealed a peak of m/z = 2532.7749 that corresponds to the exact mass of the molecular ion [M+H+Na]+ of (I₁), unambiguously confirming the structure of the targeted amide-linked macrocycle functionalized with six aldehyde groups on the periphery (FIG. 4b). The acquisition of the cleaved product was further validated with NMR studies employing ¹H NMR (FIG. 4a and FIG. 10), ¹³C NMR (FIG. 11), HSQC (FIG. 12), and HMBC (FIG. 13), experiments in DMSO-d₆.

Remarkably, the amide:aldehyde protons ratio of 2:1 in (I₁) and the absence of carboxylic acid protons signals in the ¹H and the ¹³C spectra proved the successful cleavage of the alkene bonds within the COF into aldehyde moieties, indicating the formation of one single product after the reductive ozonolysis of the (III₁). Moreover, the disappearance of the proton signals at δ = 7.62 ppm and 7.56 ppm, and the carbon signals at δ = 132.16 ppm and 128.91 ppm corresponding to the phenyl terminal groups associated with the presence of lineal oligomers backed the presence of a closed, macrocyclic structure. Functionalized macrocycle (I₁) presented a diffusion coefficient of 10-10.25 m²s⁻¹ in DOSY NMR spectrum (500 MHz, DMSO-d₆), with a calculated solvodynamic diameter of 38.80 Å, which fits to the dimensions of the macrocycle (FIG. 14).

### Example 5: Synthesis of aldehyde-functionalized macrocycle (I₂)

The co-condensation of StTA and 2,3,5,6-tetrafluoroterephthalaldehyde (4F-PDA) led to (IV₂), and the subsequent oxidation of the corresponding imine-linked COF led to (III₂). Upon the ozonolysis of this material in a mixture of DMF/THF/MeOH using reductive conditions (FIG. 3, step 3, reduction), the material could be successfully disassembled into the specific functionalized organic macrocycle (I₂)), as a highly fluorinated derivative presenting a highly hydrophobic behaviour for targeted applications such as catalysis, molecular recognition or ultrafast water permeation.

### Example 6: Difference in inner diameters of pores in macrocycles and COF precursors depending on the aldehyde linker selected

Specific functionalized macrocycles (I₁) and (I₈) obtained from (III₁), that incorporated StTA and PDA, presented an inner pore of 34.90 Å, while the implementation of the longer linkers [1,1'-Biphenyl]-4,4'-dicarbaldehyde (BPDA) and [1,1':4',1"-Terphenyl]-4,4"-dicarboxaldehyde (TPDA) during the COF synthesis led to the iso-reticular COFs (III₃) and (III₄) which, after ozonolysis, disassembled into the specific functionalized macrocycles macrocyclic analogues (I₃), (I₄), (I₇), and (I₈) with inner diameters of 42.30 Å and 50.05 Å, respectively.

### Example 7: Clip-off chemistry applied to amide-linked COFs to obtain carboxylic acid functionalized organic macrocycles

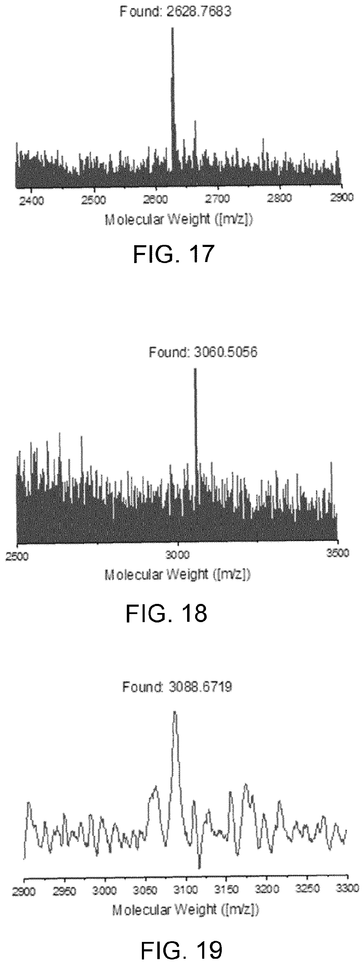

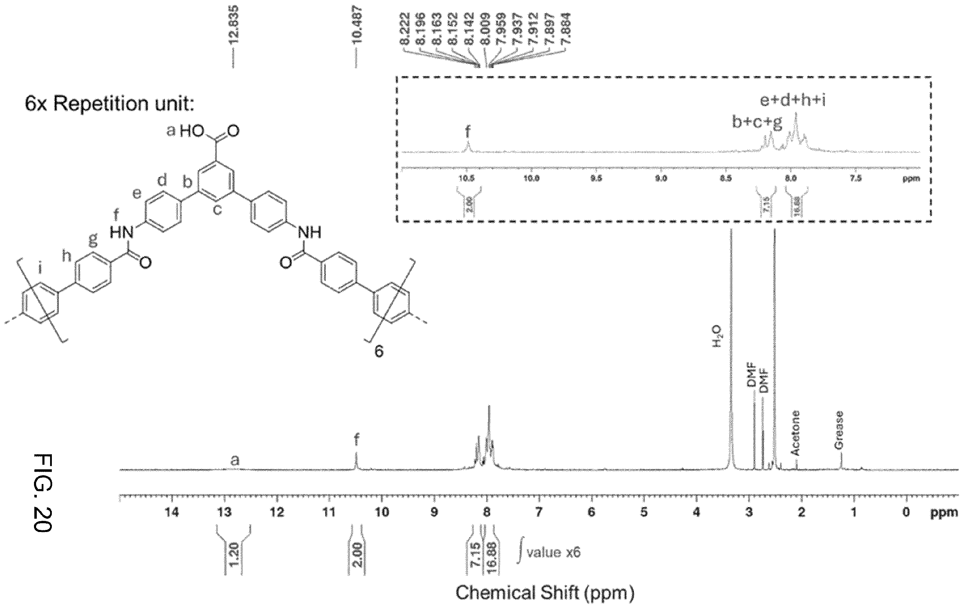

By treating the solution with an excess of Oxone^{®} after the reaction (FIG.3 step 3, oxidation), the analogous carboxylic acid functionalized macrocycles (I₈) - (I₈) were obtained, with excellent yields and high purity (FIG. 17-20). These macrocycles exhibit a significantly higher solubility profile than their aldehyde counterparts, including complete solubility in a basic methanol mixture upon deprotonation of their COOH groups.

10 mg of the corresponding amide-linked COF (III₁) - (III₄) were dispersed in a mixture of DMF/THF (Vt = 2 mL, ratio 1/1 mL) for ten minutes using an ultrasonication bath. The dispersion was cooled down to -78 °C and connected to a custom-made ozone generator. The dispersion was subsequently bubbled with ozone gas (Flow: 1 L/min, C: 50 g/N m³, approx. 3 g/h) for ten minutes. Upon completion of this time, the resulting solution was bubbled with air for five minutes. THF was evaporated from the solution under reduced pressure and, to the resulting DMF solution, Oxone^{®} (Excess: 40 mg for (III₁), 50 mg for (III₂) and (III₃), and 55 mg for (III₄)) was added. The mixture was stirred for three days at 25°C. Upon completion of the time, H₂O (3 mL) was added, and the white precipitate was stirred for two hours. The solid was washed two times with water and dried at 85 °C under reduced pressure to yield carboxylic acid functionalized macrocycles q) - t) as white solids (70-85%).

### Citation List

### Non Patent Literature

- Dichtel, W.R., et al., in "Single-Crystalline Imine-Linked Two-Dimensional Covalent Organic Frameworks Separate Benzene and Cyclohexane Efficiently", Journal of the American Chemical Society 2022, 144 (43), 19813-19824.
- Han, X.-N., et al., "Recent advances in the synthesis and applications of macrocyclic arenes", Chem. Soc. Rev., 2023, 52, 3265.
- Johnston, A. G., et al., "The Synthesis and Solubilization of Amide Macrocycles via Rotaxane Formation", J. Am. Chem. Soc. 1996, 118, 43, 10662-10663.
- Liu, W., et al., "The Synthesis of a Multiple D-A Conjugated Macrocycle and Its Application in Organic Photovoltaic", Angew Chem Int Ed Engl., Vol. 62, Issue 48, 2023.
- Marti-Centelles, V., et al., "Macrocyclization Reactions: The Importance of Conformational, Configurational, and Template-Induced Preorganization", Chem. Rev. 2015, 115, 16, 8736-8834.
- Yang, Y., et al., "Clip-off Chemistry: Synthesis by Programmed Disassembly of Reticular Materials", Angew. Chem. Int. Ed. 2022, 61, e202111228.
- Zalessky, I., et al., "A Modular Strategy for the Synthesis of Macrocycles and Medium-Sized Rings via Cyclization/Ring Expansion Cascade Reactions", J. Am. Chem. Soc. 2024, 146, 8, 5702-5711.

## Claims

1. A process for preparing a specific organic macrocycle (I) which comprises disassembling a covalent organic framework via clip-off chemistry, wherein:
the clip-off chemistry technique comprises:
submitting the covalent organic framework to a bond-breaking reaction, wherein:
the covalent organic framework comprises a dual-pore lattice forming pores by means of both resistant bonds and sensitive bonds to the bond-breaking reaction;
the bonds being in a specific position such that, when submitted to the bond-breaking reaction, the resistant bonds enable maintaining the structure of the specific macrocycle forming one of the type of pores, and the sensitive bonds enable isolating the specific macrocycle.

2. The process according to any of the claims 1, wherein the covalent organic framework is iso-reticular.

3. The process according to any of the claims 1-2, wherein the covalent organic framework is a 2D framework with dual-pore lattice.

4. The process according to claim 3, wherein the dual-pore lattice forms a 2D *Kagome* topology composed of hexagonal and trigonal pores.

5. The process according to any of the claims 1-4, wherein the dual-pore lattice is composed of combinations of tetratopic linkers and linear linkers.

6. The process according to claim 5, wherein the tetratopic linkers are tetratopic C₂h linkers and the linear linkers are aldehyde linear linkers.

7. The process according to claim 6, wherein the tetratopic C₂h linker is selected from the group consisting of (E)-3,3',5,5'-tetrakis(4-aminophenyl) stilbene, (*E*)-5'-(((4,4,"-diamino-[1,1':3',1"-terphenyl]-5'-yl)imino)methyl)-[1,1':3',1"-terphenyl]-4,4"-diamine, 5',5ʺʺ-disulfanediylbis(([1,1':3',1"-terphenyl]-4,4"-diamine)), and 5',5ʺʺ-(ethyne-1,2-diyl)bis(([1,1':3',1"-terphenyl]-4,4"-diamine)), and the aldehyde linear linker is selected from the group consisting of benzene-1,4-dicarboxaldehyde (PDA), tetrafluoroterephthalaldehyde (4F-PDA), [1,1'-Biphenyl]-4,4'-dicarbaldehyde (BPDA), and [1,1':4',1"-Terphenyl]-4,4"-dicarboxaldehyde (TPDA), forming an imine-linked covalent organic framework.

8. The process according to any of the claims 1-7, wherein the tetratopic C₂h linker is (E)-3,3',5,5'-tetrakis(4-aminophenyl) stilbene, and the aldehyde linear linker is selected from the group consisting of benzene-1,4-dicarboxaldehyde (PDA), tetrafluoroterephthalaldehyde (4F-PDA), [1,1'-Biphenyl]-4,4'-dicarbaldehyde (BPDA), and [1,1':4',1"-Terphenyl]-4,4"-dicarboxaldehyde (TPDA).

9. The process according to any of the claims 1-7, wherein the bond-breaking reaction is selected from the list consisting of ozonolysis, photothermal cleavage, redox cleavage, and pH-triggered cleavage.

10. The process according to any of the claims 1-9, wherein the bond-breaking reaction is ozonolysis, and wherein the moieties are ozone-resistant bonds and ozone-sensitive bonds.

11. The process according to claim 10, wherein the ozone-resistant moiety is an amide bond, and the ozone-sensitive moiety is an alkene bond.

12. The process according to any of the claims 10-11, wherein the process further comprises a subsequent step which comprises performing an oxidation or reduction reaction to yield a specific organic macrocycle (I) having aldehyde or carboxylic acid functional groups, respectively.

13. The process according to any of the claims 9-12, wherein the ozonolysis reaction is carried out in an appropriate solvent or solvent combination, wherein the dispersion is bubbled at a temperature ranging from -90 to 25 °C, and with a concentration of between 0.5 and 3 g/h of ozone.

14. The process according to any of the claims 1-13, wherein the covalent organic framework is disassembled into hexagonal subunits.

15. A specific functionalized organic macrocycle (I) obtainable by the process defined in any of the claims 1-14, wherein the macrocycle is selected from the following list, and having the following structure:
